# EUROPEAN PATENT APPLICATION

(11) **EP 4 450 628 A1**
(43) Date of publication of application: **23.10.2024**
(21) Application number: 22907860.5
(22) Date of filing: 09.12.2022
(51) Int. Cl.: C12N 15/63, C12N 15/113

(54) **NUCLEIC ACID EXPRESSION PLATFORM WITH INCREASED EXPRESSION EFFICIENCY**

(30) Priority: 13.12.2021 KR 20210177977
(71) Applicant: SML Biopharm Co., Ltd., Gwangmyeong-si, Gyeonggi-do 14353 (KR)
(72) Inventor: NAM, Jae Hwan, Bucheon-si Gyeonggi-do 14787 (KR); PARK, Hyung Joon, Bucheon-si Gyeonggi-do 14675 (KR); ROH, Ga Hyun, Seoul 05501 (KR)
(74) Representative: Seyer & Nobbe Patentanwälte PartmbB
(86) International application number: PCT/KR2022/020062
(87) International publication number: WO 2023/113393

(57) **Abstract**

The present disclosure relates to a nucleic acid molecule including plural translation control elements downstream inserted downstream of a coding region, and a nucleic acid expression platform or a nucleic acid expression system such as a recombinant expression vector including the nucleic acid molecule inserted therein. A target gene inserted into the coding region can be expressed efficiently using the nucleic acid molecule. It is possible to improve expression efficiency of a report gene and, an antigen and/or therapeutic protein or peptide utilizing the expression platform.

## Description

### Technical Field

The present application is supported by Ministry of Science and Information and Communication Technology of Korean Government (New/Variant Infectious Disease Response Platform Core Technology Development Business; Platform Development for New mRNA Vaccines Project; Grant No. 171158916) and by Ministry of Food and Drug Safety of Korean Government (Infectious Disease Response Innovation Technology Support Research Business; Toxic Evaluation Technology Development Research of mRNA Vaccines Project; Grant No. 1475013322).

The present disclosure relates to a nucleic acid expression platform, and more particularly, to a nucleic acid molecule with increased expression efficiency and a nucleic acid expression platform or a nucleic acid expression system such as a recombinant expression vector including the nucleic acid molecule.

### Background Art

Expression systems that can express genes of interest (GOI) to intend to express have been known since the genetic recombination technology was developed in 1970s. Generally, cell-based expression systems utilizes expression systems of microorganisms or eukaryotes, and expression systems not containing cells utilizes purified RNA polymerase, ribosome, tRNAs and ribonucleotides among those expression systems.

Proteins induced from eukaryotes experience post-translations modification such as phosphorylation, methylation and glycosylation after the expression, but microorganisms do not have such post-translational mechanism. Accordingly, eukaryotic expression system should be used in case of intending to express proteins inducible by eukaryotes.

The eukaryote induced expression system can be applied to gene therapeutics, a nucleic acid vaccine, or the like. The gene of interest consisting of an open reading frame encoding a peptide or a protein treating various diseases is inserted into the expression system in case of gene therapeutics. The gene of interest consisting of an open reading frame encoding a peptide or a protein that can be expressed to an antigen is inserted into the expression system in case of a nucleic acid vaccine. However, a nucleic acid expression system that can improve the expression efficiency of the gene of interest has been demanded by the related art.

### BRIEF SUMMARY

An object of the present disclosure is to provide a nucleic acid expression platform or a nucleic acid expression system that can improve expression efficiency of a gene of interest.

In one aspect, the present disclosure provides a nucleic acid molecule that comprises a coding region; and a translation control element linked operably to the coding region, wherein the translation control element comprises an upstream translation control element located upstream of the coding region and a downstream translation control element located downstream of the coding region, and wherein the downstream translation control element comprises plural downstream translation control elements.

In one exemplary embodiment, the downstream translation control element can comprise a first downstream translation control element and a second downstream translation control element each of which is located at the downstream of the coding region.

As an example, each of the plural downstream translation control elements (for example, the first downstream translation control element and the second downstream translation control element) can independently comprise a translation control element derived from a gene selected from the group consisting of human troponin T1 of slow skeletal type (human TNNT1), human albumin, human ferritin light chain (human FTL), human C-C motif chemokine ligand (human CCL19), human associated migratory cell protein (human AAMP), human ribosomal protein S27 (human RPS27) and human defensing alpha 5 (human DEFA5), or a transcript sequence thereof.

Each of the plural downstream translation control elements (for example, the first downstream translation control element and the second downstream translation control element) can independently comprise a translation control element selected from the group consisting of SEQ ID NO: 8, SEQ ID NO: 9, SEQ ID NO: 10, SEQ ID NO: 11, SEQ ID NO: 12, SEQ ID NO: 13 and SEQ ID NO: 14, or a transcript sequence thereof.

In one exemplary embodiment, the first downstream translation control element can comprise a translation control element derived from the human TTNT1, or a transcript sequence thereof, and the second downstream translation control element can comprise a translation control element derived from a gene selected from the group consisting of the human albumin, the human FTL, the human CCL19, the human AAMP, the human RPS27 and the human DEFA5, or a transcript sequence thereof.

In another exemplary embodiment, the first downstream translation control element can comprises a translation control element derived from the human albumin, or a transcript sequence thereof, and the second downstream translation control element can comprise a translation control element derived from the human DEFA5, or a transcript sequence thereof.

In another exemplary embodiment, the first downstream translation control element can comprise a translation control element derived from the human FTL, or a transcript sequence thereof, and the second downstream translation control element can comprise a translation control element derived from a gene selected from the group consisting of the human TNNT1, the human FTL, the human RPS27 and the human DEFA5, or a transcript sequence thereof.

In another exemplary embodiment, the first downstream translation control element can comprise a translation control element derived from the human CCL19, or a transcript sequence thereof, and the second downstream translation control element can comprise a translation control element derived from a gene selected from the group consisting of the human TNNT1, the human FTL, the human CCL19, the human RPS27 and the human DEFA5, or a transcript sequence thereof.

In another exemplary embodiment, the first downstream translation control element can comprise a translation control element derived from the human AAMP or a transcript sequence thereof, and the second downstream translation control element can comprise a translation control element derived from a gene selected from the group consisting of the human albumin, the human FTL, the human RPS27 and the human DEFA5 or a transcript sequence thereof.

In another exemplary embodiment, the first downstream translation control element can comprise a translation control element derived from the human RSP27 or a transcript sequence thereof, and the second downstream translation control element can comprise a translation control element derived from a gene selected from the group consisting of the human TNNT1, the human albumin, the human FTL, the human CCL19, the human AAMP, the human RPS27 and the human DEFA5 or a transcript sequence thereof.

In another exemplary embodiment, the first downstream translation control element can comprise a translation control element derived from the human DEFA5 or a transcript sequence thereof, and the second downstream translation control element can comprise a translation control element derived from a gene selected from the groups consisting of the human TNNT1, the human albumin, the human FTL, the human CCL19, the human AAMP and the human RPS27 or a transcript sequence thereof.

In another exemplary embodiment, the downstream translation control element can further comprise a third downstream translation control element located downstream of the second downstream translation control element.

The third downstream translation control element can comprise a translation control element derived from a gene selected from the group consisting of a human troponin T1 of slow skeletal type (human TNNT1), a human albumin, a human ferritin light chain (human FTL), a human C-C motif chemokine ligand (human CCL19), a human associated migratory cell protein (human AAMP), a human ribosomal protein S27 (human RPS27) and a human defensing alpha 5 (human DEFA5), or a transcript sequence thereof.

For example, the first downstream translation control element can comprise a translation control element derived from the human TNNT1, or a transcript sequence thereof, the second downstream translation control element can comprise a translation control element derived from the human DEFA5, or a transcript sequence thereof, and the third downstream translation control element can comprise a translation control element derived from the human FTL or the human RPS27, or a transcript sequence thereof.

In another exemplary embodiment, the first downstream translation control element can comprise a translation control element derived from the human albumin, or a transcript sequence thereof, the second downstream translation control element can comprise a translation control element derived from the human DEFA5, or a transcript sequence thereof, and the third downstream translation control element can comprise a translation control element derived from the human RPS27, or a transcript sequence thereof.

In another exemplary embodiment, the first downstream translation control element can comprise a translation control element derived from the human RPS27, or a transcript sequence thereof, the second downstream translation control element can comprise a translation control element derived from the human FTL, or a transcript sequence thereof, and the third downstream translation control element can comprise a translation control element derived from a gene selected from the group consisting of the human CCL19, the human RPS27 and the human DEFA5, or a transcript sequence thereof.

In another exemplary embodiment, the first downstream translation control element can comprises a translation control element derived from the human DEFA5, or a transcript sequence thereof, the second downstream translation control element can comprise a translation control element derived from the human TNNT1, or a transcript sequence thereof, and the third downstream translation control element can comprise a translation control element derived from the human FTL, or a transcript sequence thereof.

The upstream translation control element can comprise a translation control element derived from a gene selected from the group consisting of a human troponin T1 of slow skeletal type (human TNNT1), a human albumin, a human ferritin light chain (human FTL), a human C-C motif chemokine ligand (human CCL19), a human associated migratory cell protein (human AAMP), a human ribosomal protein S27 (human RPS27) and a human defensing alpha 5 (human DEFA5), or a transcript sequence thereof.

As an example, the upstream translation control element can comprise a translation control element selected from the group consisting of SEQ ID NO: 1, SEQ ID NO: 2, SEQ ID NO: 3, SEQ ID NO: 4, SEQ ID NO: 5, SEQ ID NO: 6 and SED ID NO: 7, or a transcript sequence thereof.

The nucleic acid molecule can be RNA.

The nucleic acid molecule can further comprise a transcription control element operably linked to the coding region.

In an alternative embodiment, the nucleic acid molecule can further comprise a polyadenylation signal sequence or a polyadenosine sequence located downstream of the downstream translation control element.

In one exemplary embodiment, the coding region can encode at least one of a reporter protein, a marker, a selection protein or a fragment thereof.

In another exemplary embodiment, the coding region can encode an antigen or a fragment thereof.

For example, the antigen can comprise a peptide of a pathogenic antigen, a tumor antigen, or a variant or a derivative thereof.

In another exemplary embodiment, the coding region can encode a peptide for disease treatment or a fragment thereof.

In another aspect, the present disclosure provides a recombinant expression vector into which the nucleic acid molecule is inserted.

In another aspect, the present disclosure provide a process of producing a protein or a peptide, the process comprises injecting the nucleic acid molecule or a recombination expression vector into which the nucleic acid molecule is inserted into a host; and expressing a protein or a peptide from the injected nucleic acid molecule in the host.

The nucleic acid expression platform of the present disclosure is designed to place the plural translation control elements a downstream of the coding region including an open reading frame of the gene of interest. An expressed transcript can be maintained stably in a body by introducing the nucleic acid expression platform. Accordingly, the final expression efficiency of the gene of interest can be improved by applying the nucleic acid expression platform of the present disclosure.

The expression efficiency of various genes of interest such as a reporter gene, an infectious antigen, a tumor antigen and/or a peptide or a protein for utilizing therapeutic purposes can be improved. The expression platform of the present disclosure can be utilized in detecting or analyzing specific materials and in preparing gene therapeutics of vaccines such as an mRNA vaccine and/or a protein subunit vaccine.

### BRIEF DESCRIPTION OF THE DRAWINGS

FIG. 1 is a schematic diagram illustrating components of a nucleic acid molecule or a polynucleotide that can efficiently express gene of interest in accordance with one embodiment of the present disclosure.
FIG. 2 is a schematic diagram illustrating components of a nucleic acid molecule or a polynucleotide that can efficiently express gene of interest in accordance with another embodiment of the present disclosure.

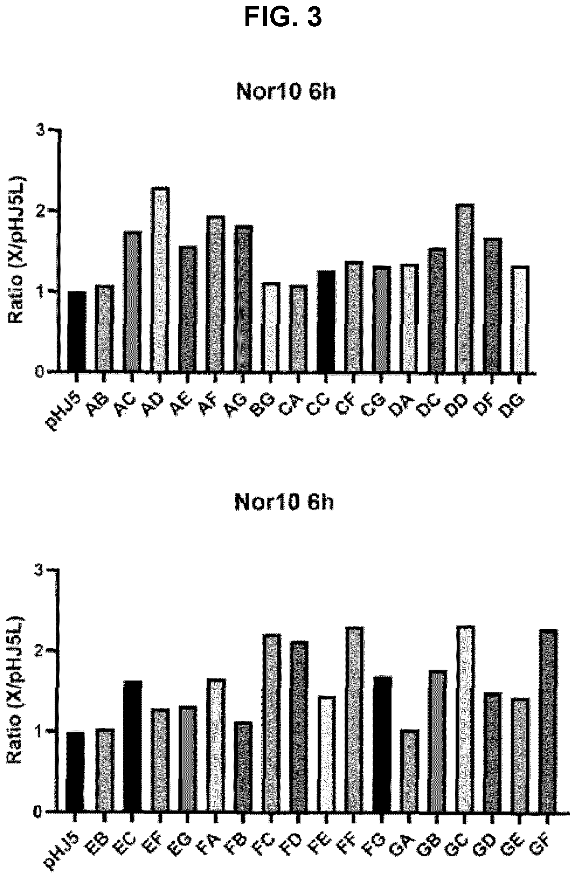

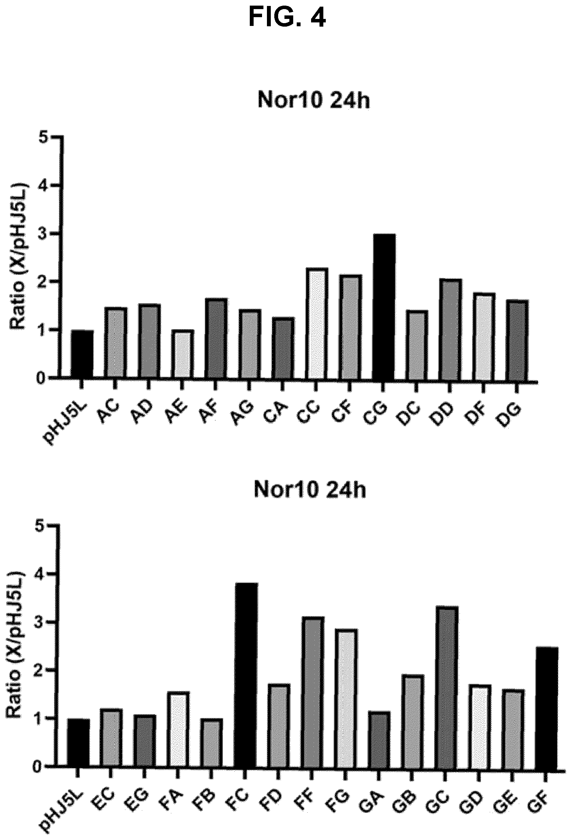

Each of FIGS. 3 and 4 is a graph illustrating the result of measuring the expression level of Renilla Luciferase at 6 hours and 24 hours, respectively, after transfecting nucleic acid molecules having a coding region encoding Renilla Luciferase into Nor10 cells of a mouse muscle-derived cell line in accordance with an Example of the present disclosure.

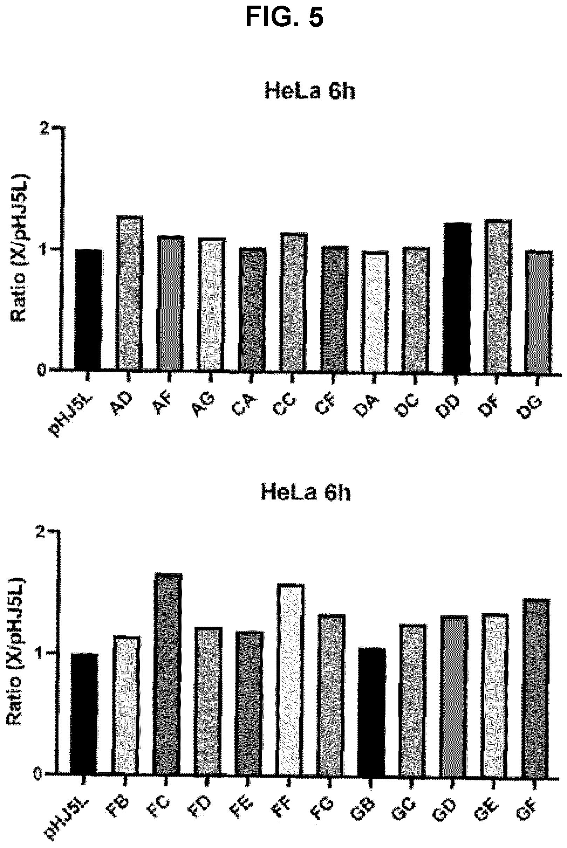

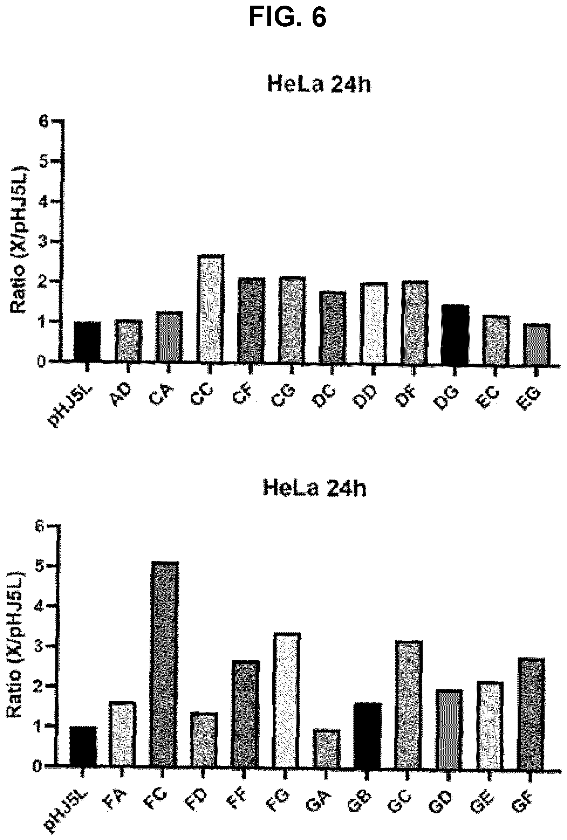

Each of FIGS. 5 and 6 is a graph illustrating the result of measuring the expression level of Renilla Luciferase at 6 hours and 24 hours, respectively, after transfecting nucleic acid molecules having a coding region encoding Renilla Luciferase into HeLa cells of a human-derived cell line in accordance with an Example of the present disclosure.

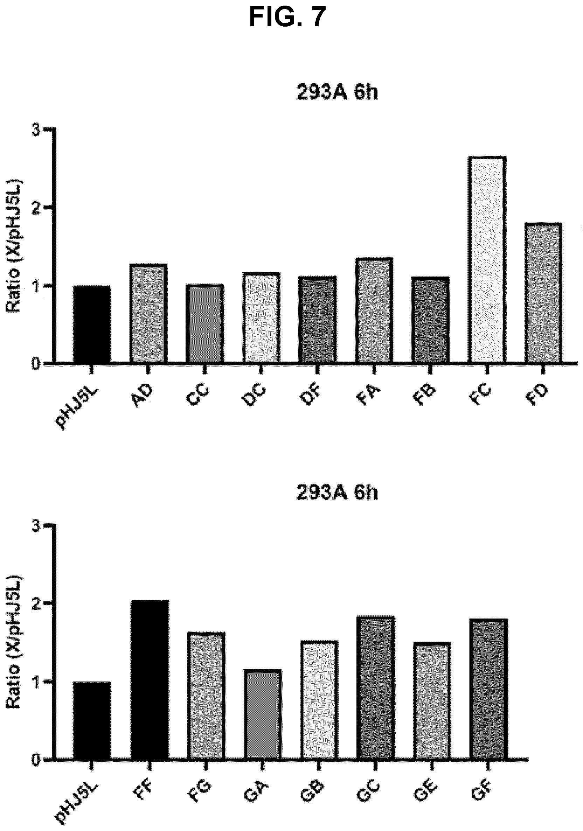

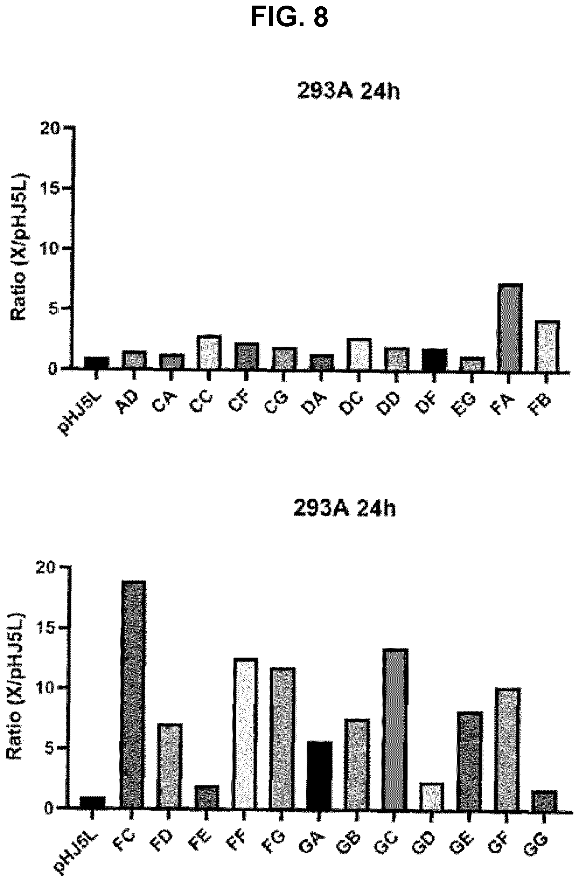

Each of FIGS. 7 and 8 is a graph illustrating the result of measuring the expression level of Renilla Luciferase at 6 hours and 24 hours, respectively, after transfecting nucleic acid molecules having a coding region encoding Renilla Luciferase into 293A cells of a human-derived cell line in accordance with an Example of the present disclosure.

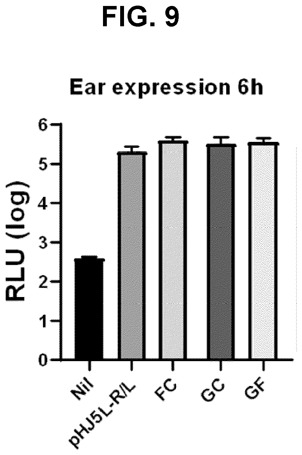

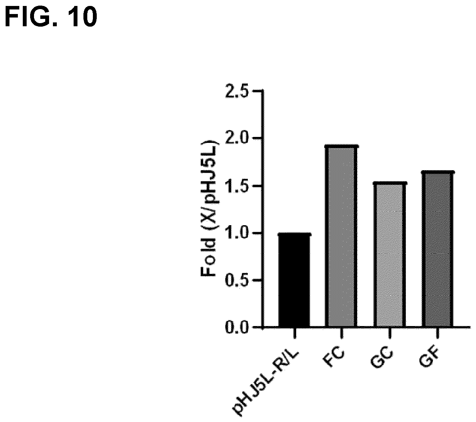

Each of FIGS. 9 and 10 is a graph illustrating the result of measuring the expression level of Renilla Luciferase at 6 hours and 24 hours, respectively, after injecting nucleic acid molecules having a coding region encoding Renilla Luciferase into a mouse ear in accordance with an Example of the present disclosure.

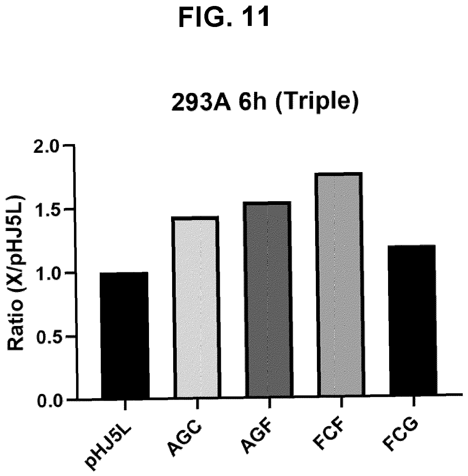

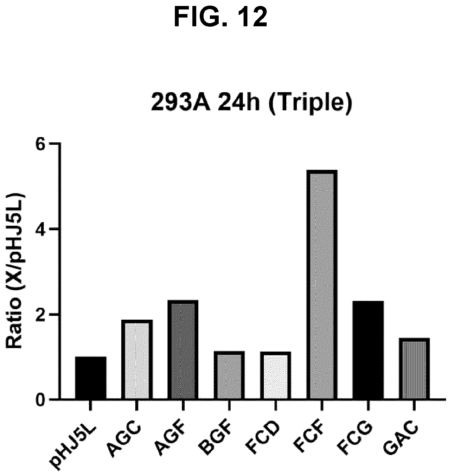

Each of FIGS. 11 and 12 is a graph illustrating the result of measuring the expression level of Renilla Luciferase at 6 hours and 24 hours, respectively, after transfecting nucleic acid molecules having a coding region encoding Renilla Luciferase into HeLa cells of a human-derived cell line in accordance with an Example of the present disclosure.

In FIGS. 3 to 12, each of "A" to G" indicates the downstream translation control sequence. The first letter indicates a first downstream translation control sequence, the second letter indicates a second downstream translation control sequence, and the third letter indicates a third downstream translation control sequence. A indicates that SEQ ID NO: 8 of a translation control sequence derived from a human TNNT1 is used, B indicates that SEQ ID NO: 9 of a translation control sequence derived from a human albumin is used, C indicates that SEQ ID NO: 10 of a translation control sequence derived from a human FTL is used, D indicates that SEQ ID NO. 11 of a translation control sequence is used, E indicates that SEQ ID NO: 12 of a translation control element derived from a human AAMP is used, F indicates that SEQ ID NO: 13 of a translation control element derived from a human RPS27 is used, and G indicates that SEQ ID NO. 14 of a translation control element derived from a human DEFA5 is used, as the downstream translation control sequence, respectively. In addition, the expression levels are compared with expression levels of peptides or proteins expressed from pHJ5 nucleic acid molecule used as Comparative Example.

### DETAILED DESCRIPTION

### Definitions

As used herein, the term "amino acid" is used in the broadest sense and is intended to include naturally occurring L-amino acids or residues thereof. Amino acid includes not only D-amino acid but also chemically-modified amino acids, for example, amino acid analogs, naturally occurring amino acids that is not typically incorporated into proteins, for example, norleucine, and chemically-synthesized compounds with amino acid-like properties known to a relevant art. For example, phenylalanine Phe or proline Pro analogs or mimetics each of which permits conformational limitations as the same as natural phenylalanine Phe or proline Pro is included in the definition of amino acid. Such analogs and mimetics are referred as "functional equivalences" of amino acids herein.

For example, synthetic peptides by standard solid-phase synthesis technique are not limited to amino acids encoded by corresponding genes, and allows the given amino acids to be substituted with much widely various ranges. Amino acids that are not encoded by the genetic code are referred as "amino acid analog" herein.

For example, amino acid analog includes, but is not limited to, 2-amino adipic acid (Aad) to glutamic acid Glu and aspartic acid Asp; 2-amino pimelic acid (Apm) to Glu and Asp; 2-amino butyric acid (Abu) to methionine Met, leucine Leu and other aliphatic amino acids; 2-amino heptanoic acid (Ahe) to Met, Leu and other aliphatic amino acids; 2-amino iso-butyric acid (Aib) to glycine Gly; cyclohexyl alanine (Cha) to valine Val, Leu and isoleucine Ile; homo arginine (Har) to arginine Arg and lysine Lys; 2,3-diamino propionic acid (Dap) to Lys, Arg and histidine His; N-ethyl glycine (EtGly) to Gly, proline Pro and alanine Ala; N-ethyl asparagine (EtAsn) to asparagine Asn and glutamine Gln; hydroxyl lysine (Hyl) to Lys; allo hydroxyl lysine (AHyl) to Lys; 3-(and 4-) hydroxyl proline (3Hyp, 4Hyp) to Pro, serine Ser and threonine Thr; allo-isoleucine (AIle) to Ile, Leu and valine Val; 4-amidino phenyl alanine to Arg; N-methyl glycine (MeGly, sarcosine) to Gly, Pro and Ala; N-methyl isoleucine (MeIle) to Ile; norvaline (Nva) to Met and other aliphatic amino acids; ornithine (Orn) to Lys, Arg and histidine His; citrulline (Cit) and methionine sulfoxide (MSO) to Thr, Asn and Gln; and N-methyl phenyl alanine (MePhe), trimethyl phenyl alanine, halo-(F-, Cl-, Br- or I-) phenyl alanine or trifluoryl phenyl alanine to Phe.

As used herein, the term `peptide' includes any of proteins, fragments of the proteins and peptides that are isolated from naturally-occurring environment or synthesized by recombinant technique or chemical synthesis. For example, the peptides of the present disclosure may comprise, but is not limited to, at least 5, for example, at least 10 amino acids.

In an exemplary embodiment, compound variants, for example, peptide variants substituted with one or more amino acids are provided. As used herein, the term "peptide variants" includes modified peptides that have one or more substitutions, deletions, addition and/or insertions of amino acids and exhibit substantially the same biological functions as the original peptide. The peptide variants should have an identity of 70% or more, preferably 90% of more, more preferably 95% or more as the original peptide. Such amino acid substituents may comprise, but is not limited to, "Conservative" amino acid substituents. Alternatively, the amino acid substituents may include non-conservative variants. In one exemplary embodiment, the polypeptide variants may have amino acid sequences different from an original amino acid sequence by substitutions, deletions, additions and/or insertions of 5 or less amino acids. Besides, peptide variants may be changed by deletions or additions of amino acids that have minimal effects upon immunogenicity, a secondary structure, and hydropathic nature of a peptide.

As used herein, the term "conservative" substitution means that there are little changes in the secondary structure in case amino acids of the polypeptides changed to other amino acids. Such amino acid variations may be obtained based upon relative similarity of side chain substituents of amino acids, for example, polarity, charge, solubility, hydrophobicity, hydrophilicity, and/or amphipathic nature.

For example, amino acids may be classified to 1) hydrophobic (methionine, alanine, valine, leucine, isoleucine), 2) neutral hydrophilic (cysteine, serine, threonine, asparagine, glutamine), 3) acidic (aspartic acid, glutamic acid), 4) basic (histidine, lysine, arginine), 5) residues having influence on the chain directions (glycine, proline), and 6) aromatic (tryptophan, tyrosine, phenylalanine) based upon the common side chains properties. Conservative variation will accompany an exchange of one member in each of the classes for another member in the same class.

It has been known that any of arginine, lysine and histidine has positively charged residue; alanine, glycine and serine has similar sizes; phenylalanine, tryptophan and tyrosine has similar shapes by analyzing the size, shapes and kinds the amino acids side chain substituents. Accordingly, each of arginine, lysine and histidine; each of alanine, glycine and serine; and each of phenylalanine, tryptophan and tyrosine may be biologically functional equivalents by analysis with regard to the sizes, shapes and kinds of the amino acid side chain substituents.

Hydropathic index may be considered in introducing variations. Each amino acid is given hydropathic index based upon its own hydrophobicity and charge: Isoleucine (+4.5); Valine (+4.2); Leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamic acid (-3.5); glutamine (-3.5); aspartic acid (-3.5); asparagine (-3.5); lysine (-3.9); and arginine (-4.5).

Hydropathic index of amino acids is very important in bestowing peptides or proteins with interactive biological functions. It has been known that similar biological activities may be maintained in only substituting amino acids with other amino acids having similar hydropathic indices. In case of introducing variations considering the hydropathic index, reciprocal substitutions among amino acids having hydropathic index value differences within preferably ±2, more preferably ±1, further more preferably ±0.5 are done.

Also, it is well known that reciprocal substitutions among amino acids having similar hydrophilicity induce proteins having equivalently biological activities. As disclosed in US Patent No. 4,554,101, following hydrophilicity value are accorded to each amino acid residue: Arginine (+3.0); lysine (+3.0); aspartic acid (+3.0 ±1); glutamic acid (+3.0+1); serine (+0.3); asparagine (+0.2); glutamine (+0.2); glycine (0); threonine (-0.4); proline (-0.5±1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); and tryptophan (-3.4). In case of introducing variations considering the hydrophilicity value, reciprocal substitutions among amino acids having hydrophilicity value differences within preferably ±2, more preferably ±1, further more preferably ±0.5 are done.

Amino acid exchanges in proteins that do not generally modify the molecular activities are known in the art (See, H. Neurath, R.L. Hill, The proteins, Academic Press, New York, 1979). The most commonly occurred exchanges are inter-exchange of amino acid residues between Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly.

Generally, the peptides (including fusion proteins) and polynucleotides described herein may be isolated. "Isolated" peptides or polynucleotides are separated from the original environment. For example, naturally occurring proteins are isolated by removing whole or part of co-existent material in a natural state. Such polypeptides should have purity of 90% or more, preferably 95% or more, more preferably 99% or more. Polynucleotides are isolated by cloning in the vectors. The recombinant peptides encoded by nucleotide sequences mentioned herein may be prepared easily by known methods irrespective of any vectors among the known vectors. The expression may be performed within an appropriate host cell transfected with the expression vector including nucleic acid sequences encoding the recombinant proteins. The appropriate host cell includes prokaryotes, yeast and eukaryotes. As an example, eukaryotes-derived cell lines such as an insect cell or a mammalian cell (*e.g.,* Cos or CHO and the like) may be utilized as the host cell.

As used herein, the term "polynucleotide" or "nucleic acid" are used inter-changeably, refers to polymers of any lengths of nucleotides, and includes comprehensibly DNA (i.e. cDNA) and RAN molecules. "Nucleotide", which is a subunit of nucleic acid molecules, may comprises, but is not limited to, a deoxyribonucleotide, a ribonucleotide, a modified nucleotide or a base, analogs thereof, and/or any substrates that can be incorporated into polynucleotides by DNA or RNA polymerase or synthetic reactions. Polynucleotide may comprise modified nucleotides, analogues having modified bases and/or polysaccharides such as methylated nucleotides and analogues thereof. The polynucleotide may comprise the modified nucleotide, an analog with a modified sugar or a base, for example, a methylated nucleotide and analogs thereof.

Some variations in nucleotides do not result in variations of peptides or proteins. Such nucleic acid variants may include any nucleic acid molecules having codons encoding functionally equivalent or identical amino acids (for example, 6 codons encodes Arg or Ser by the degeneracy of codons) or encoding biologically equivalent amino acids. On the other hand, other variations in nucleotides may induce changes in peptides or proteins. In spite of variations causing changes of amino acids of proteins, it is possible to obtain variant proteins that show substantially the same activities as the proteins of the present disclosure.

A person having ordinary skill in the art will appreciate that peptides and nucleic acids herein is not limited to the peptides and the nucleic acids described in the Sequence Listing. Rather, it is intended that peptides or the proteins as well as the nucleic acid molecules encoding the peptides or the proteins of the present disclosure may comprise any amino acid sequences or nucleotide sequences that has substantially the same biological functions such as vaccine and/or adjuvant. For example, the coding region operably linked to the expression control element and/or biologically equivalents thereof that can be included in the recombinant proteins/peptides expressed by the coding region can be any polynucleotide with varying base sequences and/or proteins/peptides with varying amino acid sequences exhibiting equivalent biological functions.

Considering the variations having the above biologically equivalent activities, the nucleic acid molecules encoding the peptide and/or the protein in accordance with the present disclosure can be interpreted to comprise any sequences showing substantial identity as the sequences described in the Sequence Listing. The substantial identity can mean any sequences having at least 61% homology, preferably at least 70% homology, more preferably at least 80% homology, and most preferably at least 90% homology in case analyzing the sequences aligned by using algorithms conventionally used in the art. The alignment process for comparing sequences is known to in the art.

As used herein, the term "vector" means a construct or a vehicle that can be transfected or delivered into the host cells, and enables one or more genes of interest (or target genes of target sequences) to be expressed within the cells. For example, the vector may include viral vectors, DNA or RNA expression vectors, plasmid, cosmid or phage vectors, DNA or RNA expression vectors linked to CCA (cationic condensing agents), DNA or RNA expression vectors packaged with liposomes, and particular eukaryotic cells such as producer cell.

As used herein, the term "expression control/regulation sequence" or "expression control/regulation element" may mean nucleic acid sequences regulating or controlling transcriptional processes of the nucleic acid molecules and/or translational processes of the nucleic acid molecules of transcripts. As used herein, the term "transcription control/regulation sequence" or "transcription control/regulation element" means that nucleic acid sequences regulating or controlling the transcriptional process of the nucleic acid molecules. For example, the transcription control element may comprise promoters such as a constitutive promoter or an inducible promoter, enhancers, and the likes.

As used herein, the term "translation control/regulation sequence" or "translation control/regulation element" may be used to indicate nucleic acid sequences regulating or controlling the translational processes of the nucleic acid molecules of transcripts to the proteins or peptides. Each of the expression control sequence/element, the transcription control sequence/element and the translation control sequence/element is operably linked to the target sequences to be expressed, for example, to be transcribed or translated.

As used herein, the term "operably linked" means a functional linkage between expression control sequence (e.g., promoters, signal sequences, ribosome linkage sites, transcription termination sequence, and the likes) and other nucleic acid sequences so that the expression control sequence may regulate transcriptions and/or translations of the other nucleic acid sequences.

### Nucleic Acid Molecule

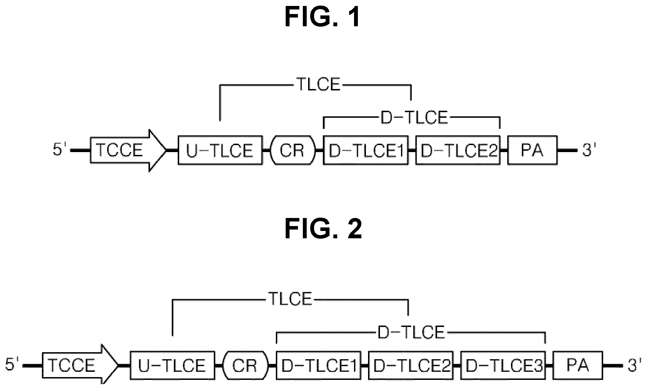

The present disclosure relates to a nucleic acid molecule that improves expression efficiency of a coding region operably linked to an expression control element and a nucleic acid-based expression platform or expression system including the nucleic acid molecule. FIG. 1 is a schematic diagram illustrating components of a nucleic acid molecule that can efficiently express proteins or peptides causing infectious diseases in accordance with one embodiment of the present disclosure.

As illustrated in FIG. 1, the nucleic acid molecule can comprise a translation control element TCLE including nucleotide sequences having a translation initiation activity, and a coding region CR operably linked to the translation control element TLCE and including an open reading frame (ORF) of gene of interest (GOI).

Alternatively or additionally, the nucleic acid molecule can further comprise at least one nucleotide sequences of a transcription control element TCCE operably linked to the coding region CR, and/or a polyadenylation signal sequence or a polyadenosine sequence PA located downstream of the translation control element TLCE.

The translation control element TLCE can comprises nucleotide sequences having the translation initiation activity operably linked to the coding region CR into which gene of interest as the ORF is inserted. For example, the translation control element TLCE can comprise an upstream translation control element U-TLCE located upstream of the coding region CR, and/or a downstream translation control element D-TLCE located downstream of the coding region CR.

In other words, the upstream translation control element U-TLCE can be located at the upstream of the coding region CR and the downstream translation control element D-TLCE can be located at the downstream of the coding region CR. The coding region CR can be located between the upstream translation control element U-TLCE and the downstream translation control element D-TLCE, and can constituted target sequence (TS) including the open reading frame of the gene of interest.

In one embodiment, the upstream transcription control element U-TLCE can be a 5'-untranslatied region (5'-UTR) with a cap dependent translation initiation activity or a part or a fragment thereof.

Most eukaryotic mRNAs have 7-methyl-guanosine as a cap at 5' terminus thereof. Translation initiation complex including various proteins recognizes the cap located at 5' terminus and proceeds to AUG of an initiation codon to initiate protein synthesis. The cap structure located at 5' terminus of the mRNAs enables the protein synthesis to initiate and to block destruction of mRNA structure from nuclease activity.

For example, the first translation process *in vitro* can be performed by treating a plasmid DNA (pDNA) with restriction endonuclease to linearize the pDNA, and attaching m7G(5')-ppp(5')G (referred to regular cap analog) to mRNA prepared using an appropriate RNA polymerase to create capped mRNA. Optionally, there is a method of performing *in vitro* transcription without the cap analog and then performing a cap reaction using a commercially available vaccinia virus capping enzyme. In this case, an 'anti-reverse' cap analog (ARCA) that prevents the reverse action of the cap can be used. When the ARCA is introduced, only 3'-O-methylation of methylated guanosine can bind to the nucleotide of unmethylated guanosine.

As an example, the modified 5'-cap structure includes Cap1 (ribose methylation within nucleotides adjacent to m7G), Cap2 (ribose methylation in nucleotide located at second downstream of m7G), Cap3 (ribose methylation in nucleotide located at third downstream of m7G), Cap4 (ribose methylation in nucleotide located at fourth downstream of m7G), ARCA or modified ARCA (e.g., phosphothioate modified ARCA), inosine, N1-methyl-guanosine, 2'-fluoro-guanosine, 7-diaza-guanosine, 8-oxo-guanosine, 2-amino-guanosine, LNA-guanosine and 2-azido-guanosie.

For example, the upstream translation control element U-TLCE and/or the downstream translation control element D-TLCE can comprise nucleotide sequences with the cap-dependent translation initiation activity derived from animals, for example, mammalians, particularly Primate, more particularly human, or a transcript sequence thereof. The upstream translation control element U-TLCE is an element or an area where the translation initiation complex is bound in the translation process of the peptide and/or protein encoded in the coding region CR and can be cis-acting nucleotide sequences inducing the translation of the coding region.

When the upstream translation control element U-TLC is nucleotide sequences with the cap-dependent translation initiation activity or a fragment thereof, the nucleic acid molecule can comprise the downstream translation control element D-TLCE located at the downstream of the coding region CR. The nucleic acid molecule including both the upstream translation control element U-TLCE and the downstream translation control element D-TLCE enables the expression of the open reading frame (ORF) constituting the coding region CR to be improved. In other words, the upstream translation control element U-TLCE and the downstream translation control element D-TLCE can improve the expression efficiency of the open reading frame of the gene of interest (GOI) or a transcript sequence thereof constituting the coding region CR, and play an important role in maintaining stably mRNA of the transcript in the cell without destroying.

In one exemplary embodiment, the upstream translation control element U-TLCE can comprise, but is not limited to, a translation control element derived from a gene selected from the group consisting of a human troponin T1 of slow skeletal type (human TNNT1), a human albumin (human ALB), a human ferritin light chain (human FTL), a human C-C motif chemokine ligand 19 (human CCL19), a human associated migratory cell protein (human AAMP), a human ribosomal protein S27 (human RPS27) and a human defensing alpha 5 (human DEFA5).

For example, the upstream translation control element U-TLCE derived from the human TNNT1 can comprise a translation control element of SEQ ID NO: 1 or a transcript sequence thereof. The upstream translation control element U-TLCE derived from the human ALB can comprise a translation control element of SEQ ID NO: 2 or a transcript sequence thereof. The upstream translation control element U-TLCE derived from the human FTL can comprise a translation control element of SEQ ID NO: 3 or a transcript sequence thereof. The upstream translation control element U-TLCE derived from the human CCL19 can comprise a translation control element of SEQ ID NO: 4 or a transcript sequence thereof. The upstream translation control element U-TLCE derived from the human AAMP can comprise a translation control sequence of SEQ ID NO: 5 or a transcript sequence thereof. The upstream translation control element U-TLCE derived from the human RPS27 can comprise a translation control element of SEQ ID NO: 6 or a transcript sequence thereof. The upstream translation control element U-TLCE derived from the human DEFA5 can comprise a translation control element of SEQ ID NO: 7 or a transcript sequence thereof. However, the upstream translation control element U-TLCE is not limited to a particular nucleotide sequence of a transcript thereof.

The downstream translation control element D-TLCE can comprise a first downstream translation control element T-TLCE1 and a second downstream translation control element D-TLCE2 located sequentially at the downstream of the coding region CR. The first downstream translation control element D-TLCE1 can be identical to or different from the second downstream translation control element D-TLCE2.

As an example, each of the first downstream translation control element D-TLCE1 and the second downstream translation control element D-TLCE2 can independently comprise a downstream translation control element derived from any gene selected from the group consisting of the human TNNT1, the human ALB, the human FTL, the human CCL19, the human AAMP, the human RPS27 and the human DEFA5.

For example, each of the first and second downstream translation control elements D-TLCE1 and D-TLCE2 derived from the human TNNT1 can comprise a translation control element of SEQ ID NO: 8 or a transcript sequence thereof. Each of the first and second downstream translation control elements D-TLCE1 and D-TLCE2 derived from the human ALB can comprise a translation control element of SEQ ID NO: 9 or a transcript sequence thereof. Each of the first and second downstream translation control elements D-TLCE1 and D-TLCE2 derived from the human FTL can comprise a translation control element of SEQ ID NO: 10 or a transcript sequence thereof. Each of the first and second downstream translation control elements D-TLCE1 and D-TLCE2 derived from the human CCL19 can comprise a translation control element of SEQ ID NO: 11 or a transcript sequence thereof. Each of the first and second downstream translation control elements D-TLCE1 and D-TLCE2 derived from the human AAMP can comprise a translation control element of SEQ ID NO: 12 or a transcript sequence thereof. Each of the first and second downstream translation control elements D-TLCE1 and D-TLCE2 derived from the human RPS27 can comprise a translation control element of SEQ ID NO: 13 or a transcript sequence thereof. Each of the first and second downstream translation control elements D-TLCE1 and D-TLCE2 derived from the human DEFA5 can comprise a translation control element of SEQ ID NO: 14 or a transcript sequence thereof. However, the first and second downstream am translation control elements D-TLCE1 and D-TLCE2 are not limited to a particular nucleotide sequence of a transcript thereof.

In one exemplary embodiment, the first downstream translation control element D-TLCE1 can comprise a translation control element derived from the human TTNT1, or a transcript sequence thereof, and the second downstream translation control element D-TLCE2 can comprise a translation control element derived from a gene selected from the group consisting of the human ALB, the human FTL, the human CCL19, the human AAMP, the human RPS27 and the human DEFA5, or a transcript sequence thereof. For example, when the first downstream translation control element D-TLCE1 comprises the downstream translation control element derived from the human TNNT1 or the transcript thereof, the second downstream translation control element D-TLCE2 can be derived from the downstream control element selected from the group consisting of the human FTL, the human CCL19 and the human DEFA5, or a transcript sequence thereof.

In an alternative embodiment, when the first downstream translation control element D-TLCE1 comprises the downstream translation control element derived from the human TNNT1 or the transcript thereof, the second downstream translation control element D-TLCE2 can comprise the downstream translation control element derived from the human FTL or the human CCL19 or a transcript sequence thereof. In another alternative embodiment, when the first downstream translation control element D-TLCE1 comprises the downstream translation control element derived from the human TNNT1 or the transcript thereof, the second downstream translation control element D-TLCE2 can comprise the downstream translation control element derived from the human CCL19 or the human RPS27, or a transcript sequence thereof.

In another embodiment, the first downstream translation control element D-TLCE1 can comprises a translation control element derived from the human albumin, or a transcript sequence thereof, and the second downstream translation control element D-TLCE2 can comprise a translation control element derived from the human DEFA5, or a transcript sequence thereof.

In another exemplary embodiment, the first downstream translation control element D-TLCE1 can comprise a translation control element derived from the human FTL, or a transcript sequence thereof, and the second downstream translation control element D-TLCE2 can comprise a translation control element derived from a gene selected from the group consisting of the human TNNT1, the human FTL, the human RPS27 and the human DEFA5, or a transcript sequence thereof. For example, when the first downstream translation control element D-TLCE1 comprises the downstream translation control element derived from the human FTL, or a transcript sequence thereof, the second downstream translation control element D-TLCE2 can comprise the downstream translation control element derived from the gene selected from the group consisting of the human TNNT1, the human FTL, the human RPS27 and the human DEFA5, or a transcript sequence thereof.

In an alternative embodiment, when the first downstream translation control element D-TLCE1 comprises the downstream translation control element derived from the human FTL or the transcript thereof, the second downstream translation control element D-TLCE2 can comprise the downstream translation control element derived from the gene selected from the group consisting of the human TNNT1, the human FTL and the human RPS27, or a transcript sequence thereof. In another alternative embodiment, when the first downstream translation control element D-TLCE1 comprises the downstream translation control element derived from the human FTL or the transcript thereof, the second downstream translation control element D-TLCE2 can comprise the downstream translation control element derived from the human FTL, or a transcript sequence thereof.

In another exemplary embodiment, the first downstream translation control element D-TLCE1 can comprise a translation control element derived from the human CCL19, or a transcript sequence thereof, and the second downstream translation control element D-TLCE2 can comprise a translation control element derived from a gene selected from the group consisting of the human TNNT1, the human FTL, the human CCL19, the human RPS27 and the human DEFA5, or a transcript sequence thereof. For example, when the first downstream translation control element D-TLCE1 comprises the downstream translation control element derived from the human CCL19 or a transcript sequence thereof, the second downstream translation control element D-TLCE2 can comprise the downstream translation control element derived from the gene selected from the group consisting of the human FTL, the human CCL19, the human RPS27 and the human DEFA5, or a transcript thereof.

In an alternative embodiment, when the first downstream translation control element D-TLCE1 comprises the downstream translation control element derived from the human CCL19 or the transcript thereof, the second downstream translation control element D-TLCE2 can comprise the downstream translation control element derived from the human FTL or the human RPS27, or a transcript sequence thereof. In another alternative embodiment, when the first downstream translation control element D-TLCE1 comprises the downstream translation control element derived from the human CCL19 or the transcript thereof, the second downstream translation control element D-TLCE2 can comprise the downstream translation control element derived from the human CCL19, or a transcript sequence thereof. In another alternative embodiment, when the first downstream translation control element D-TLCE1 comprises the downstream translation control element derived from the human CCL19 or the transcript thereof, the second downstream translation control element D-TLCE2 can comprise the downstream translation control element derived from the human CCL19 or the human RPS27, or a transcript sequence thereof.

In another alternative embodiment, when the first downstream translation control element D-TLCE1 comprises the downstream translation control element derived from the human CCL19 or the transcript thereof, the second downstream translation control element D-TLCE2 can comprise the downstream translation control element derived from the human FTL or the human CCL19, or a transcript sequence thereof.

In another exemplary embodiment, the first downstream translation control element T-TLCE1 can comprise a translation control element derived from the human AAMP or a transcript sequence thereof, and the second downstream translation control element D-TLCE2 can comprise a translation control element derived from a gene selected from the group consisting of the human albumin, the human FTL, the human RPS27 and the human DEFA5 or a transcript sequence thereof.

In another exemplary embodiment, the first downstream translation control element D-TLCE1 can comprise a translation control element derived from the human RSP27 or a transcript sequence thereof, and the second downstream translation control element D-TLCE2 can comprise a translation control element derived from a gene selected from the group consisting of the human TNNT1, the human albumin, the human FTL, the human CCL19, the human AAMP, the human RPS27 and the human DEFA5 or a transcript sequence thereof. For example, when the first downstream translation control element D-TLCE1 comprises the downstream translation control element derived from the human RPS27 or the transcript thereof, the second downstream translation control element D-TLCE2 can comprise the downstream translation control element derived from the gene selected from the group consisting of the human TNNT1, the human FTL, the human CCL19, the human AAMP, the human RPS27 and the human DEFA5, or a transcript sequence thereof.

In an alternative embodiment, when the first downstream translation control element D-TLCE1 comprises the downstream translation control element derived from the human RPS27 or the transcript thereof, the second downstream translation control element D-TLCE2 can comprise the downstream translation control element derived from the gene selected from the group consisting of the human FTL, the human CCL19, the human RPS27 and the human DEFA5, or a transcript sequence thereof. In another alternative embodiment, when the first downstream translation control element D-TLCE1 comprises the downstream translation control element derived from the human RPS27 or the transcript thereof, the second downstream translation control element D-TLCE2 can comprise the downstream translation control element derived from the gene selected from the group consisting of the human FTL, the human RPS27 and the human DEFA5, or a transcript sequence thereof.

In an alternative embodiment, when the first downstream translation control element D-TLCE1 comprises the downstream translation control element derived from the human RPS27 or the transcript thereof, the second downstream translation control element D-TLCE2 can comprise the downstream translation control element derived from the gene selected from the group consisting of the human TNNT1, the human FTL, the human RPS27 and the human DEFA5, or a transcript sequence thereof. In another alternative embodiment, when the first downstream translation control element D-TLCE1 comprises the downstream translation control element derived from the human RPS27 or the transcript thereof, the second downstream translation control element D-TLCE2 can comprise the downstream translation control element derived from the human RPS27 or the human DEFA5, or a transcript sequence thereof. In another alternative embodiment, when the first downstream translation control element D-TLCE1 comprises the downstream translation control element derived from the human RPS27 or the transcript thereof, the second downstream translation control element D-TLCE2 can comprise the downstream translation control element derived from the human FTL, or a transcript sequence thereof.

In another exemplary embodiment, the first downstream translation control element D-TLCE1 can comprise a translation control element derived from the human DEFA5 or a transcript sequence thereof, and the second downstream translation control element D-TLCE2 can comprise a translation control element derived from a gene selected from the groups consisting of the human TNNT1, the human albumin, the human FTL, the human CCL19, the human AAMP and the human RPS27 or a transcript sequence thereof. For example, when the first downstream translation control element D-TLCE1 comprises the downstream translation control element derived from the human DEFA5 or the transcript thereof, the second downstream translation control element D-TLCE2 can comprise the downstream translation control element derived from the gene selected from the group consisting of the human TNNT1, the human ALB, the human FTL, the human AAMP and the human RPS27, or a transcript sequence thereof.

In an alternative embodiment, when the first downstream translation control element D-TLCE1 comprises the downstream translation control element derived from the human DEFA5 or the transcript thereof, the second downstream translation control element D-TLCE2 can comprise the downstream translation control element derived from the gene selected from the group consisting of the human ALB, the human FTL, the human AAMP and the human RPS27, or a transcript sequence thereof. In another alternative embodiment, when the first downstream translation control element D-TLCE1 comprises the downstream translation control element derived from the human DEFA5 or the transcript thereof, the second downstream translation control element D-TLCE2 can comprise the downstream translation control element derived from the gene selected from the group consisting of the human ALB, the human FTL and the human RPS27, or a transcript sequence thereof.

In an alternative embodiment, when the first downstream translation control element D-TLCE1 comprises the downstream translation control element derived from the human DEFA5 or the transcript thereof, the second downstream translation control element D-TLCE2 can comprise the downstream translation control element derived from the gene selected from the group consisting of the human FTL, the human CCL19, the human AAMP and the human RPS27, or a transcript sequence thereof. In another alternative embodiment, when the first downstream translation control element D-TLCE1 comprises the downstream translation control element derived from the human DEFA5 or the transcript thereof, the second downstream translation control element D-TLCE2 can comprise the downstream translation control element derived from the human FTL or the human RPS27, or a transcript sequence thereof.

The coding region CR is not particularly limited if the coding region CR includes the open reading frame (ORF) of the intended gene of interest (GOI). In one embodiment, the coding region CR can comprise an open reading frame (ORF) of the nucleotide sequence encoding at least one of a reporter protein or fragments thereof, a marker or selection protein or fragments thereof, an antigen or fragments thereof and a protein for curing disease or fragments thereof, or the transcript thereof.

As an example, the coding region CR can comprise the open reading frame (ORF) encoding the reporter protein/reporter peptide such as luciferase protein (e.g., Renilla luciferase), Green Fluorescent Protein (GFP), Enhanced Green Fluorescent Protein (EGFP), glucuronidase such as beta-glucuronidase, and/or galactosidase such as beta-galactosidase, or fragments thereof.

In another embodiment, the coding region CR can comprise an open reading frame encoding marker or selection protein/peptide such as globin proteins such as alpha-globin and/or beta-globin, galactokinase and/or xanthine guanine phosphoribosyl transferase, or fragments thereof. In addition, any open reading frame encoding other reporter protein/peptides and/or marker or selection proteins/peptides can be inserted into the coding region CR.

In another embodiment, the antigenic gene inserted into the coding region CR can comprise an open reading frame (ORF) encoding a protein/peptide including a pathogenic antigen or fragments thereof, a tumor antigen, variants or derivatives thereof.

The pathogenic antigen is induced from the organism, particularly mammalians, more particularly pathogenic organisms causing immune responses in a human, particularly, bacterial, viruses or protozoan (multi-cellular) pathogenic organisms. More particularly, the pathogenic antigen can be a surface antigen, for example, proteins (or fragments thereof, for example, external part of the surface antigen), presented on the surface of viruses, bacteria or protozoan organisms.

For example, the pathogenic antigen can be a peptide or protein antigen derived from pathogens associated with infectious disease. More particularly, the pathogenic antigen can be induced from, but is not limited to, a nucleotide sequence or a fragment thereof encoding Acinetobacter baumannii, Anaplasma genus, Anaplasma phagocytophilum, Ancylostoma braziliense, Ancylostoma duodenale, Arcanobacterium haemolyticum, Ascaris lumbricoides, Aspergillus genus, Astroviridae, Babesia genus, Bacillus anthracis, Bacillus cereus, Bartonella henselae, BK virus, Blastocystis hominis, Blastomyces dermatitidis, Bordetella pertussis, Borrelia burgdorferi, Borrelia genus, Borrelia spp, Brucella genus, Brugia malayi, Bunyaviridae family, Burkholderia cepacia and other Burkholderia spp, Burkholderia mallei, Brukholderia psudomallei, Caliciviridae family, Campylobacter genus, Candida albicans, Candida spp, Chlamydia trachomatis, Chlamydophila pneumoniae, Chlamydophila psittaci, CJD prion, Clonorchis sinensis, Clostridium botulinum, Clostridium difficile, Clostridium perfringens, Clostridium perfringens, Clostridium spp, Clostridium tetani, Coccidioides spp, coronaviruses, Corynebacterium diphtheria, Coxiella burnetii, Crimean-Congo hemorrhagic fever virus, Cryptococcus neoformans, Cryptosporidium genus, Cytomegalovirus (CMV), Dengue viruses (DEN-1, DEN-2, DEN-3 and DEN-4), Dientamoeba fragilis, Ebolavirus (EBOV), Echinococcus genus, Ehrlichia chaffeensis, Ehrlichia ewingii, Ehrlichia genus, Entamoeba histolytica, Enterococcus genus, Enterovirus genus, Enteroviruses, particularly Coxsackie A virus) and EV71, Epidermophyton spp, Epstein-Barr Virus (EBV), Escherichia coli O157:H7, O111 and O104:H4, Fasciola hepatica and Fasciola gigantica, FFI prion, Filarioidea superfamily, Flaviviruses, Francisella tularensis, Fusobacterium genus, Geotrichum candidum, Giardia intestinalis, Gnathostoma spp, GSS prion, Guanarito virus, Haemophilus ducreyi, Haemophilus influenza, Helicobacter pylori, Henipavirus (Hendra virus Nipah virus), Hepatitis A virus, Hepatitis B virus (HPV), Hepatitis C virus (HCV), Hepatitis D virus, Hepatitis E virus, Herpes simplex viruses 1 and 2 (HSV-1 and HSV-2), Histoplasma capsulatum, HIV (Human immunodeficiency virus), Hortaea werneckii, Human bocavirus (HBoV), Human herpesvirus 6 (HHV-6) and Human herpesvirus 7 (HHV-7), Human metapneumovirus (hMPV), Human papillomavirus (HPV), Human parainfluenza viruses (HPIV), Japanese encephalitis virus, JC virus, Junin virus, Kingella kingae, Klebsiella granulomatis, Kuru prion, Lassa virus, Legionella pneumophila, Leishmania genus, Leptospira genus, Listeria monocytogenes, Lymphocytic choriomeningitis virus (LCMV), Machupo virus, Malassezia spp, Marburg virus, Measles virus, Metagonimus yokagawai, Microsporidia phylum, Molluscum contagiosum virus (MCV), Mumps virus, Mycobacterium leprae and Mycobacterium lepromatosis, Mycobacterium tuberculosis, Mycobacterium ulcerans, Mycoplasma pneumoniae, Naegleria fowleri, Necator americanus, Neisseria gonorrhoeae, Neisseria meningitides, Nocardia asteroids, Nocardia spp, Onchocerca volvulus, Orientia tsutsugamushi, Orthomyxoviridae family (influenza), Paracoccidioides brasiliensis, Paragonimus spp, Paragonimus westermani, Parvovirus B19, Pasteurella genus, Plasmodium genus, Pneumocystis jirovecii, Poliovirus, Rabies virus, Respiratory syncytial virus (RSV), Rhinovirus, Rickettsia akari, Rickettsia genus, Rickettsia prowazekii, Rickettsia rickettsia, Rickettsia typhi, Rift Valley fever virus, Rotavirus, Rubella virus, Sabia virus, Salmonella genus, Sarcoptes scabiei, SARS coronavirus, Schistosoma genus, Severe fever with thrombocytopenia syndrome virus (SFTSV), Shigella genus, Sin Nombre virus, Hantavirus, Sporothrix schenckii, Staphylococcus genus, Streptococcus agalactiae, Streptococcus pneumoniae, Streptococcus pyogenes, Strongyloides stercoralis, Taenia genus, Taenia solium, Tickborne encephalitis virus (TBEV), Toxocara canis or Toxocara cati, Toxoplasma gondii, Treponema pallidum, Trichinella spiralis, Trichomonas vaginalis, Trichophyton spp, Trichuris trichiura, Trypanosoma brucei, Trypanosoma cruzi, Ureaplasma urealyticum, Varicella zoster virus (VZV), Variola major or Variola minor), vCJD prion, Venezuelan equine encephalitis virus, Vibrio cholera, West Nile virus, Western equine encephalitis virus, Wuchereria bancrofti, Yellow fever virus, Yersinia enterocolitica, Yersinia pestis, and Yersinia pseudotuberculosis.

In an alternative embodiment, the coding region CR can comprise an open reading frame (ORF) encoding a protein or a peptide of a tumor antigen or a fragment thereof, variants or derivatives thereof. The tumor antigen can be a melanocyte-specific antigen, testicular tumor antigen or a tumor-specific antigen, for example, CT-X antigen, non-X CT antigen, a binding partner for CT-X antigen or a binding partner for non-X CT antigen or a tumor-specific antigen, or a tumor-specific antigen, variants or derivatives of the tumor antigen.

The open reading frame (ORF) encoding such tumor antigen can comprise, but is not limited to, nucleotide sequence encoding 5T4, 707-AP, 9D7, AFP, AlbZIP HPG1, alpha-5-beta-1-integrin, alpha-5-beta-6-integrin, alpha-actini-4/m, alpha-methyl acyl-coenzyme A racemase, ART-4, ARTC1/m, B7H4, BAGE-1, BCL-2, bcr/abl, beta-catenin/m, BING-4, BRCA1/m, BRCA2/m, CA 15-3/CA 27-29, CA 19-9, CA72-4, CA125, calreticulin, CAMEL, CASP-8/m, cathepsin B, carhepsin L, CD19, CD20, CD22, CD25, CDE30, CD33, CD4, CD52, CD55, CD56, CD80, CDC27/m, CDK4/m, CDKN2A/m, CEA, CLCA2, CML28, CML66, COA-1/m, coactosin-like protein, collage XXIII, COX-2, CT-9/BRD6, Cten, cyclin B1, cyclin D1, cyp-B, CYPB1, DAM-10, DAM-6, DEKCAN, EFTUD2/m, EGFR, ELF2/m, EMMPRIN, EpCam, EphA2, EphA3, ErbB3, ETV6-AML1, EZH2, FGF-5, FN, Frau-1, G250, GAGE-1, GAGE-2, GAGE-3, GAGE-4, GAGE-5, GAGE-6, GAGE7b, GAGE-8, GDEP, GnT-V, gp100, GPC3, GPNMB/m, HAGE, HAST-2, hepsin, Her2/neu, HERV-K-MEL, HLA-A*0201-R17I, HLA-A11/m, HLA-A2/m, HNE, Homeobox NKX3.1, HOM-TES-14/SCP-1, HOM-TES-85, HPV-E6, HPV-E7, HSP70-2M, HST-2, hTERT, iCE, IGF-1R, IL-13Ra2, IL-2R, IL-5, immature laminin receptor, kallikrein-2, krein-4, Ki67, KIAA0205, KIAA0205/m, KK-LC-1, K-Ras/m, LAGE-A1, LDLR-FUT, MAGE-A1, MAGE-A2, MAGE-A3, MAGE-A4, MAGE-A6, MAGE-A9, MAGE-A10, MAGE-A12, MAGE-B1, MAGE-B2, MAGE-B3, MAGE-B4, MAGE-B5, MAGE-B6, MAGE-B10, MAGE-B16, MAGE-B17, MAGE-C1, MAGE-C2, MAGE-C3, MAGE-D1, MAGE-D2, MAGE-D4, MAGE-E1, MAGE-E2, MAGE-F1, MAGE-H1, MAGEL2, mammaglobin A, MART-1/melan-A, MART-2, MART-2/m, matrix protein 22, MC1R, M-CSF, ME 11m, mesothelin, MG50/PXDN, MMP11, MN/CA IX-antigen, MRP-3, MUC-1, MUC-2, MUM-11m, MUM-2/m, MUM-3/m, myosin class I/m, NA88-A, N-acetylgluocosminyl transferase-V, Neo-PAP, Neo-PAP/m, NFYC/m, NGEP, NMP22, NPM/ALK, N-Ras/m, NSE, NY-ESO-1, NY-ESO-B, OA1, OFA-iLRP, OGT, OGT/m, OS-9, OS-9/m, Osteocalcin, Osteopontin, p15, p190 minor bcr-abl, p53, p53/m, PAGE-4, PAI-1, PAI-2, PAP, PART-1, PATE, PDEF, Pim-1-kinase, Pin-1, Pml/PAR alpha, POTE, PRAME, PRDX5/m, prostain, proteinase-3, PSA, PSCA, PSGR, PSM, PSMA, PTPRK/m, RAGE-1, RBAF600/m, RHAMM/CD168, RU1, RU2, S-100, SAGE, SART-1, SART-2, SART-3, SCC, SIRT2/m, Sp17, SSX-1, SSX-2/HOM-MEL-40, SSX-4, STAMP-1, STEAP-1, Survivin, Survivin-2B, SYT-SSX-1, SYT-SSX-2, TA-90, TAG-72, TARP, TEL-AML1, TGF beta, TGF beta RII, TGM-4, TPI/m, TRAG-3, TRG, TRP-1, TRP-2/6b, TRP/INT2, TRP-p8, Tyrosinase, UPA, VEGFR1, VEGFR-2/FLK-1, WT1, immunoglobulin genotype of lymphoid blood cell or T-cell receptor genotype of lymphoid blood cell, or fragments, variants or derivatives thereof, or a transcript thereof.

In another embodiment, the coding region CR can comprise an open reading frame (ORF) encoding a protein/peptide of therapeutic protein/peptide, or a fragment, a variant or a derivative thereof.

Therapeutic protein may play an important role in producing therapy that can modify or treat genetic disorders, destroy cancer cells or pathogen-infected cells, treat immune-system diseases and treat metabolic or endocrine diseases. As an example, the therapeutic proteins can be utilized as various purposes including treatments of various diseases such as infectious diseases, neoplasm (e.g., cancer or tumor diseases), diseases of blood or blood-producing organs, endocrine diseases, nutrient and metabolic diseases, nervous system diseases, circulatory system diseases, respiratory system diseases, digestive system diseases, skin and subcutaneous tissue diseases, musculoskeletal diseases, connective tissue diseases and genitourinary system irrespective of genetic or acquired disorders.

For example, the peptides or proteins used for treating metabolic or endocrine disorders (specific disease for therapeutic protein) may comprise, is not limited to, Acid sphingomyelinase (Niemann-Pick disease), Adipotide (obesity), Agalsidase-beta (human galactosidase A) (Fabry disease; prevents accumulation of lipids that could lead to renal and cardiovascular complications), Alglucosidase (Pompe disease (glycogen storage disease type II)), alpha-galactosidase A (alpha-GAL A, Agalsidase alpha) (Fabry disease), alpha-glucosidase (Glycogen storage disease (GSD), Morbus Pompe), alpha-L-iduronidase (mucopolysaccharidoses (MPS), Hurler syndrome, Scheie syndrome), alpha-N-acetylglucosaminidase (Sanfilippo syndrome), Amphiregulin (cancer, metabolic disorder), Angiopoietin ((Ang1, Ang2, Ang3, Ang4, ANGPTL2, ANGPTL3, ANGPTL4, ANGPTL5, ANGPTL6, ANGPTL7) (angiogenesis, stabilize vessels), Betacellulin (metabolic disorder), Beta-glucuronidase (Sly syndrome), Bone morphogenetic protein BMPs (BMP1, BMP2, BMP3, BMP4, BMP5, BMP6, BMP7, BMP8a, BMP8b, BMP10, BMP15) (regenerative effect, bone-related conditions, chronic kidney disease (CKD)), CLN6 protein (CLN6 disease - Atypical Late Infantile, Late Onset variant, Early Juvenile, Neuronal Ceroid Lipofuscinoses (NCL)), Epidermal growth factor (EGF) (wound healing, regulation of cell growth, proliferation, and differentiation), Epigen (metabolic disorder), Epiregulin (metabolic disorder), Fibroblast Growth Factor (FGF, FGF-1, FGF-2, FGF-3, FGF-4, FGF-5, FGF-6, FGF-7, FGF-8, FGF-9, FGF-10, FGF-11, FGF-12, FGF-13, FGF-14, FGF-16, FGF-17, FGF-17, FGF-18, FGF-19, FGF-20, FGF-21, FGF-22, FGF-23) (wound healing, angiogenesis, endocrine disorders, tissue regeneration), Galsulphase (Mucopolysaccharidosis VI), Ghrelin (irritable bowel syndrome (IBS), obesity, Prader-Willi syndrome, type II diabetes mellitus), Glucocerebrosidase (Gaucher's disease), GM-CSF (regenerative effect, production of white blood cells, cancer), Heparin-binding EGF-like growth factor (HB-EGF) (wound healing, cardiac hypertrophy and heart development and function), Hepatocyte growth factor HGF (regenerative effect, wound healing), Hepcidin (iron metabolism disorders, Beta-thalassemia), Human albumin (Decreased production of albumin (hypoproteinaemia), increased loss of albumin (nephrotic syndrome), hypovolaemia, hyperbilirubinaemia), Idursulphase (Iduronate-2-sulphatase) (Mucopolysaccharidosis II (Hunter syndrome)), Integrins αVβ3, αVβ5 and β5β1 (Bind matrix macromolecules and proteinases, angiogenesis), Iuduronate sulfatase (Hunter syndrome), Laronidase (Hurler and Hurler-Scheie forms of mucopolysaccharidosis I), N-acetylgalactosamine-4-sulfatase (rhASB; galsulfase, Arylsulfatase A (ARSA), Arylsulfatase B (ARSB)) (arylsulfatase B deficiency, Maroteaux-Lamy syndrome, mucopolysaccharidosis VI), N-acetylglucosamine-6-sulfatase (Sanfilippo syndrome), Nerve growth factor (NGF, Brain-Derived Neurotrophic Factor (BDNF), Neurotrophin-3 (NT-3), and Neurotrophin 4/5 (NT-4/5) (regenerative effect, cardiovascular diseases, coronary atherosclerosis, obesity, type 2 diabetes, metabolic syndrome, acute coronary syndromes, dementia, depression, schizophrenia, autism, Rett syndrome, anorexia nervosa, bulimia nervosa, wound healing, skin ulcers, corneal ulcers, Alzheimer's disease), Neuregulin (NRG1, NRG2, NRG3, NRG4) (metabolic disorder, schizophrenia), Neuropilin (NRP-1, NRP-2) (angiogenesis, axon guidance, cell survival, migration), Obestatin (irritable bowel syndrome (IBS), obesity, Prader-Willi syndrome, type II diabetes mellitus), Platelet Derived Growth factor (PDGF (PDFF-A, PDGF-B, PDGF-C, PDGF-D) (regenerative effect, wound healing, disorder in angiogenesis, Arteriosclerosis, Fibrosis, cancer), TGF beta receptors (endoglin, TGF-beta 1 receptor, TGF-beta 2 receptor, TGF-beta 3 receptor) (renal fibrosis, kidney disease, diabetes, ultimately end-stage renal disease (ESRD), angiogenesis), Thrombopoietin (THPO) (Megakaryocyte growth and development factor (MGDF)) (platelets disorders, platelets for donation, recovery of platelet counts after myelosuppressive chemotherapy), Transforming Growth factor (TGF; (TGF-a, TGF-beta (TGF-beta1, TGF-beta2, and TGF-beta3)) (regenerative effect, wound healing, immunity, cancer, heart disease, diabetes, Marfan syndrome, Loeys-Dietz syndrome), VEGF (VEGF-A, VEGF-B, VEGF-C, VEGF-D, VEGF-E, VEGF-F and PIGF) (regenerative effect, angiogenesis, wound healing, cancer, permeability), Nesiritide (Acute decompensated congestive heart failure), Trypsin (Decubitus ulcer, varicose ulcer, debridement of eschar, dehiscent wound, sunburn, meconium ileus), adrenocorticotrophic hormone (ACTH) ("Addison's disease, Small cell carcinoma, Adrenoleukodystrophy, Congenital adrenal hyperplasia, Cushing's syndrome, Nelson's syndrome, Infantile spasms), Atrial-natriuretic peptide (ANP) (endocrine disorders), Cholecystokinin (diverse), Gastrin (hypogastrinemia), Leptin (Diabetes, hypertriglyceridemia, obesity), Oxytocin (stimulate breastfeeding, non-progression of parturition), Somatostatin (symptomatic treatment of carcinoid syndrome, acute variceal bleeding, and acromegaly, polycystic diseases of the liver and kidney, acromegaly and symptoms caused by neuroendocrine tumors), Vasopressin (antidiuretic hormone) (diabetes insipidus), Calcitonin (Postmenopausal osteoporosis, Hypercalcaemia, Paget's disease, Bone metastases, Phantom limb pain, Spinal Stenosis), Exenatide (Type 2 diabetes resistant to treatment with metformin and a sulphonylurea), Growth hormone (GH), somatotropin (Growth failure due to GH deficiency or chronic renal insufficiency, Prader-Willi syndrome, Turner syndrome, AIDS wasting or cachexia with antiviral therapy), Insulin (Diabetes mellitus, diabetic ketoacidosis, hyperkalaemia), Insulin-like growth factor 1 IGF-1 (Growth failure in children with GH gene deletion or severe primary IGF1 deficiency, neurodegenerative disease, cardiovascular diseases, heart failure), Mecasermin rinfabate, IGF-1 analog (Growth failure in children with GH gene deletion or severe primary IGF1 deficiency, neurodegenerative disease, cardiovascular diseases, heart failure), Mecasermin, IGF-1 analog (Growth failure in children with GH gene deletion or severe primary IGF1 deficiency, neurodegenerative disease, cardiovascular diseases, heart failure), Pegvisomant (Acromegaly), Pramlintide (Diabetes mellitus, in combination with insulin), Teriparatide (human parathyroid hormone residues 1-34) (Severe osteoporosis), Becaplermin (Debridement adjunct for diabetic ulcers), Dibotermin-alpha (Bone morphogenetic protein 2) (Spinal fusion surgery, bone injury repair), Histrelin acetate (gonadotropin releasing hormone; GnRH) (Precocious puberty), Octreotide (Acromegaly, symptomatic relief of VIP-secreting adenoma and metastatic carcinoid tumours), and Palifermin (keratinocyte growth factor; KGF) (Severe oral mucositis in patients undergoing chemotherapy, wound healing), (in brackets is the particular disease for which the therapeutic protein is used in the treatment).

The therapeutic the protein (specific disease for protein treatment) with regard to the treatment of blood disorders, diseases of the circulatory system, diseases of the respiratory system, cancer or tumor diseases, infectious diseases or immune-deficiencies may comprise, but is not limited to, Alteplase (tissue plasminogen activator; tPA) (Pulmonary embolism, myocardial infarction, acute ischaemic stroke, occlusion of central venous access devices), Anistreplase (Thrombolysis), Antithrombin III (AT-III) (Hereditary AT-III deficiency, Thromboembolism), Bivalirudin (Reduce blood-clotting risk in coronary angioplasty and heparin-induced thrombocytopaenia), Darbepoetin-alpha (Treatment of anaemia in patients with chronic renal insufficiency and chronic renal failure (+/- dialysis)), Drotrecogin-alpha (activated protein C) (Severe sepsis with a high risk of death), Erythropoietin, Epoetin-alpha, erythropoetin, erthropoyetin (Anaemia of chronic disease, myleodysplasia, anaemia due to renal failure or chemotherapy, preoperative preparation), Factor IX (Haemophilia B), Factor VIIa (Haemorrhage in patients with haemophilia A or B and inhibitors to factor VIII or factor IX), Factor VIII (Haemophilia A), Lepirudin (Heparin-induced thrombocytopaenia), Protein C concentrate (Venous thrombosis, Purpura fulminans), Reteplase (deletion mutein of tPA) (Management of acute myocardial infarction, improvement of ventricular function), Streptokinase (Acute evolving transmural myocardial infarction, pulmonary embolism, deep vein thrombosis, arterial thrombosis or embolism, occlusion of arteriovenous cannula), Tenecteplase (Acute myocardial infarction), Urokinase (Pulmonary embolism), Angiostatin (Cancer), Anti-CD22 immunotoxin (Relapsed CD33⁺ acute myeloid leukaemia), Denileukin diftitox (Cutaneous T-cell lymphoma (CTCL)), Immunocyanin (bladder and prostate cancer), MPS (Metallopanstimulin) (Cancer), Aflibercept (Non-small cell lung cancer (NSCLC), metastatic colorectal cancer (mCRC), hormone-refractory metastatic prostate cancer, wet macular degeneration), Endostatin (Cancer, inflammatory diseases like rheumatoid arthritis as well as Crohn's disease, diabetic retinopathy, psoriasis, and endometriosis), Collagenase (Debridement of chronic dermal ulcers and severely burned areas, Dupuytren's contracture, Peyronie's disease), Human deoxy-ribonuclease I, dornase (Cystic fibrosis; decreases respiratory tract infections in selected patients with FVC greater than 40% of predicted), Hyaluronidase (Used as an adjuvant to increase the absorption and dispersion of injected drugs, particularly anaesthetics in ophthalmic surgery and certain imaging agents), Papain (Debridement of necrotic tissue or liquefication of slough in acute and chronic lesions, such as pressure ulcers, varicose and diabetic ulcers, burns, postoperative wounds, pilonidal cyst wounds, carbuncles, and other wounds), L-Asparaginase (Acute lymphocytic leukaemia, which requires exogenous asparagine for proliferation), Peg-asparaginase (Acute lymphocytic leukaemia, which requires exogenous asparagine for proliferation), Rasburicase (Paediatric patients with leukaemia, lymphoma, and solid tumours who are undergoing anticancer therapy that may cause tumour lysis syndrome), Human chorionic gonadotropin (HCG) (Assisted reproduction), Human follicle-stimulating hormone (FSH) (Assisted reproduction), Lutropin-alpha (Infertility with luteinizing hormone deficiency), Prolactin (Hypoprolactinemia, serum prolactin deficiency, ovarian dysfunction in women, anxiety, arteriogenic erectile dysfunction, premature ejaculation, oligozoospermia, asthenospermia, hypofunction of seminal vesicles, hypoandrogenism in men), alpha-1-Proteinase inhibitor (Congenital antitrypsin deficiency), Lactase (Gas, bloating, cramps and diarrhea due to inability to digest lactose), Pancreatic enzymes (lipase, amylase, protease) (Cystic fibrosis, chronic pancreatitis, pancreatic insufficiency, post-Billroth II gastric bypass surgery, pancreatic duct obstruction, steatorrhoea, poor digestion, gas, bloating), Adenosine deaminase (pegademase bovine, PEG-ADA) (Severe combined immunodeficiency disease due to adenosine deaminase deficiency), Abatacept (Rheumatoid arthritis (especially when refractory to TNFa inhibition)), Alefacept (Plaque Psoriasis), Anakinra (Rheumatoid arthritis), Etanercept (Rheumatoid arthritis, polyarticular-course juvenile rheumatoid arthritis, psoriatic arthritis, ankylosing spondylitis, plaque psoriasis, ankylosing spondylitis), Interleukin-1 (IL-1) receptor antagonist, Anakinra (inflammation and cartilage degradation associated with rheumatoid arthritis), Thymulin (neurodegenerative diseases, rheumatism, anorexia nervosa), TNF-alpha antagonist (autoimmune disorders such as rheumatoid arthritis, ankylosing spondylitis, Crohn's disease, psoriasis, hidradenitis suppurativa, refractory asthma), Enfuvirtide (HIV-1 infection), and Thymosin al (Hepatitis B and C).

There is no limitation in the length of the ORFs in the coding region CR, and the expression efficiency depending on the ORF length is not considered in developing a nucleic acid molecule, a recombinant expression vector, and nucleic acid vaccine for treatment or prevention using the molecule. Codon usage is not considered in developing human vaccines or gene therapies because codon usage basis in human has not influence on common peptides/proteins expression significantly, while codon usage may have an influence on the expression of proteins/peptides in various species. But, it may be necessary that start codon have Kozak sequence and nucleotides adjacent to termination codon may be optimized. If necessary, the third codon among GOI or its transcript mRNA codon to be expressed may be changed "G/C" without changing amino acid so that mRNA may have improved stability.

Alternatively or additionally, other nucleotides can be inserted into the nucleic acid molecule so that the expression efficiency of the coding region CR as the ORF can be further increased. As an example, the nucleic acid molecule can have a transcription control element TCCE promoting the transcription of the target sequence adjacently to the translation control element TLCE, for example, to the upstream translation control element U-TLCE. For example, the transcription control element TCCE can be located upstream of the upstream translation control element U-TLCE. Such a transcription control element TCCE is not particularly limited, and can be described with regard to the following recombinant expression vector in order to avoid duplicative descriptions.

In addition, a nucleotide sequence that can induce the expression of the open reading frame (ORF) comprising a genetic sequence of the transcript sequence thereof constituting the coding region CR can be further inserted into the nucleic acid molecule, in addition to the above translation control sequence TLCE, the coding region CR and the transcription control element TCCE. In one exemplary embodiment, Kozak sequence can be inserted between the upstream translation control element TLCE and the start codon of the coding region CR.

In addition, a polyadenylation signal sequence and/or a polyadenosine sequence PA can be inserted at the downstream of the coding region CR, more particularly, at the downstream of the second downstream translation control element D-TLCE2. The polyadenylation signal sequence of the polyadenosine sequence PA can stabilize the transcribed nucleic acid molecule and can further improve the translation efficiency of the open reading frame (ORF) including the genetic sequence of transcript sequence in the coding region CR.

For example, when the nucleic acid molecule of the present disclosure is present as a transcript of mRNA, the polyadenosine sequence PA can be nucleotides of about 25 to about 400, for example, about 30 to about 400, about 50 to about 250 or about 60 to 250 adenosines.

In another exemplary embodiment, the nucleic acid molecule of the present disclosure is present as a DNA type, the polyadenylation signal sequence PA can be located at downstream of the second downstream translation control element D-TLCE2. As an example, the polyadenylation signal sequence PA can be derived, but is not limited to, SV40, human growth factor (hGH), bovine growth hormone (BGH), rabbit beta-globin (rbGlob), and the likes.

Alternatively or additionally, the polyadenylation signal sequence or the polyadenosine sequence PA can include another signal sequences or linker sequences such as 5'-GATCATCAGT'-3' between two nucleotides that each of which comprises multiple adenosines, for example, about 25 to about 400, about 30 to about 400, about 50 to about 250 or about 60 to about 250 or transcripts thereof.

The nucleic acid molecule can comprise at least one Cloning Site, preferably Multiple Cloning Site (MCS) for inserting the coding region CR therein. The at least one Cloning Site can comprise at least one restriction endonuclease recognition site and/or site cleaved by at least one restriction endonuclease. In one embodiment, the restriction endonuclease may comprise artificially engineered restriction endonuclease (e.g. zinc finger nuclease or restriction endonuclease based on DNA binding site of TAL effector or PNA-based PNAzymes) as well as naturally-occurring endonuclease found in bacterial or archaebacteria.

For example, the naturally-occurring restriction endonuclease may be classified into 1) Type I endonuclease (cuts sites spaced apart from recognition site and requires ATP, S-adenosyl-L-methionine and Mg²⁺), 2) Type II endonuclease (cuts specific sites within or spaced apart from recognition site and most requires Mg²⁺), 3) Type III endonuclease (cuts specific site spaced apart from recognition site and requires only ATP without hydrolysis of ATP), 4) Type IV endonuclease (targets modified sites such as methylation, hydroxyl methylation or glucosyl-hydroxyl methylation), and 5) Type V endonuclease (e.g. CRISPR cas9-mRNA complex), and the likes.

For example, the site recognized and/or cleaved by the following restriction endonuclease can be included, but is not limited to, in the Multiple Cloning Site: 5'-ATCGAT-3' (AngI), 5'-AGGCCT-3' (AatI), 5'-TGATCA-3' (AbaI), 5'-GGATCC-3' (BamH), 5'-GCAGC(N)8-3' (BbvI, 5'-(N)₁₀CGA(N)₆TGC(N)₁₂-3' (BcgI), 5'-(N)₈GAG(N)₅CTC(N)₁₃-3' (BplI), 5'-GTCTC(N)-3' (BsmAI, Alw26I), 5'-ACTGGN-3' (BsrI), 5'-ATCGAT-3' (Clal), 5'-CTCTTCN-3' (EarI), 5'-CTGAAA(N)₁₆-3' (Eco57I), 5'-GAATTC-3' (EcoRI), 5'-CCWGG-3' (EcoRII, w indicates A or T), 5'-GATATC-3' (EcoRV), 5'-GGATG(N)₉-3' (FokI), 5'GGCC-3' (HaeIII), 5'-AAGCTT-3' (HindIII), 5'-CCGG-3' (HpaIII), 5'-GGTGA(N)₈-3' (HphI), 5'-GGTACC-3' (KpnI), 5'-GATC-3' (Mbol), 5'-ACGCGT-3' (MluI), 5'-GCCGGC-3' (NaeI), 5'-GATATG-3' (NdeII), 5'-GCCGGC-3' (NgoMIV), 5'-CATG-3' (NlaIII), 5'-GCGGCCGC-3' (NotI), 5'-TTAATTAA-3' (PacI), 5'-CTGCAG-3' (PstI), 5'-GAGCTC-3' (SacI), 5'-CCGCGG-3' (SacII), 5'-GTCGAC-3' (Sail), 5'-GCATC(N)₅-3' (SfaNI), 5'-CCCGGG-3' (SmaI), 5'-TCGA-3' (TaqI), 5'-TCTAGA-3' (Xbal), 5'-CGCGAG-3' (XhoI), 5'-CGATCG-3' (PvuI), and combinations thereof.

In another exemplary embodiment, the MCS can consist of SEQ ID NO: 19.

The nucleic acid molecule can have a DNA type or an RNA type. In one embodiment, the nucleic acid molecule of the present disclosure can have the RNA type. When the nucleic acid molecule has the RNA type, for example, when the coding region CR comprises the ORF transcript encoding the proteins or peptides above, the RNA type nucleic acid molecule can have advantages over the DNA type nucleic acid molecule.

Firstly, the RNA type nucleic acid molecule does not need to enter the nucleus of the host cells for transcription into mRNA, unlike the DNA type nucleic acid molecule. Accordingly, there is low risk of potentially inducing mutations in the RNA type nucleic acid molecule, and the RNA type nucleic acid molecule is stable owing to rapid dissociation within the body. It is not possible for the RNA type nucleic acid molecule to be integrated into the host chromosome within the nucleus. Anti-biotic resistance genes, which are selection markers used to selectively produce the nucleic acid molecule in host cells, are unnecessary for the production of the RNA type nucleic acid molecule. The RNA does not induce long-time persistence genetic transformation because the RNA has a shorter half-life than DNA. While the nucleic acid molecule delivered into the cells is activated for short times to express the target peptide/protein, and then destroyed with several days through enzymatic reactions, the immune response specific to the originally expressed target peptide/protein is remained.

Secondly, the RNA type nucleic acid molecules can induce a desired *in vivo* immune response even when used in relatively small amounts compared to the DNA type nucleic acid molecules. As described above, the RNA type nucleic acid molecule only passes though the cell membrane without the need to enter the nucleus unlike the DNA type nucleic acid molecule. Accordingly, a relatively small amount of RNA type nucleic acid molecule compared to the DNA type nucleic acid molecule can express the target peptide.

Thirdly, the entire manufacturing processes can be artificially controlled in preparing the RNA type nucleic acid molecules, so that the RNA type nucleic acid molecules can be safely produced in small-scale GMP (good manufacturing practice) production facilities without the risk of biological contamination. There is no need to directly treat the infectious agents in producing the RNA nucleic acid molecules. Instead, only the nucleic acid sequences of the neutralizing antibody-inducing part (neutralizing epitope) of the infectious agent to be expressed is artificially synthesized, and performed *in vitro* transcription (IVT) in mass producing the mRNA.

Fourthly, the RNA type nucleic acid molecules can induce a stronger immune response than naked DNA nucleic acid molecules. The RNA type nucleic acid molecules itself can produce a complex antigen within the cell, which can function as an ideal adjuvant by accessing MHC (Major histocompatibility complex) class II of antigen-presenting cells. Additionally, multiple antigens to induce immune responses can be produced simultaneously, mixed, and then immunized. There are no special restrictions on the gene length of the antigen to be expressed, which can increase the applicability and simplicity of producing the mRNA type nucleic acid molecules.

Fifthly, RNA type nucleic acid molecule can be produced easily compared to the DNA type nucleic acid molecule. As described above, since the RNA type nucleic acid molecule can be synthesized through the IVT process, there is no need to directly deal with live viruses or pathogenic micro-organisms used in the production of general attenuate or killed vaccines, and there is no need to culture host cells such as yeast, *E.coli,* or insect cells, which should be used to produce recombinant proteins/peptides. Massive amount of RNA nucleic acid molecule can be produced with *in vitro* transcription (IVT). Recently, reagents related to IVT reactions, particularly, DNA-dependent RNA polymerase have been improved, making it possible to rapidly produce large amounts of RNA within 1 to 2 weeks using a small amount of DNA template. In addition, plural antigens intending to induce immune responses can be prepared simultaneously, and can be immunized after mixing the plural antigens, and there is no limitation in lengths of the antigens to be expressed, so that the RNA type nucleic acid molecule can be prepared with ease.

In another embodiment, nucleic acid molecule can comprise three or more downstream translation control elements. FIG. 2 is a schematic diagram illustrating components of a nucleic acid molecule or a polynucleotide that can efficiently express gene of interest in accordance with another embodiment of the present disclosure.

As illustrated in FIG. 2, the nucleic acid molecule comprises a translation control element TLCE including nucleotide sequences having translation initiation activity, and a coding region CR operably linked to the translation control element TLCE and comprising an open reading frame (ORF) of a gene of interest (GOI), optionally, a transcription control element TCCE located at upstream of the translation control element TLCE and/or a polyadenylation signal sequence or a polyadenosine sequence PA. The translation control element TLCE comprises an upstream translation control element U-TLCE located at upstream of the coding region CR, and a downstream translation control element D-TLCE located at downstream of the coding region CR. In this embodiment, the downstream translation control element D-TLCE comprises a first downstream translation control element D-TLCE1, a second downstream translation control element D-TLCE2 and a third downstream translation control element D-TLCE3 arranged sequentially between the coding region CR and the polyadenylation signal sequence PA. The nucleic acid molecule illustrated in FIG. 2 can have identical configurations to the nucleic acid molecule illustrated in FIG. 1 except that the downstream translation control element D-TLCE further comprises the third downstream translation control element D-TLCE3.

As an example, each of the first downstream translation control element D-TLCE1, the second downstream translation control element D-TLCE2 and the third downstream translation control element D-TLCE3 can independently comprise a downstream translation control element selected from the group consisting of a human troponin T1 of slow skeletal type (TNNT1), a human albumin, a human ferritin light chain (FTL), a human C-C motif chemokine ligand 19 (CCL19), a human associated migratory cell protein (AAMP), a human ribosomal protein 27 (RPS27) and a human defensing alpha 5 (DEFA5), or a transcript sequence thereof.

For example, each of the first downstream translation control element D-TLCE1, the second downstream translation control element D-TLCE2 and the third downstream translation control element D-TLCE3 can independently comprise a downstream translation control element selected from the group consisting of SEQ ID NO: 8, SEQ ID NO: 9, SEQ ID NO: 10, SEQ ID NO: 11, SEQ ID NO: 12, SEQ ID NO: 13 and SEQ ID NO: 14, or a transcript sequence thereof.

In one exemplary embodiment, the first downstream translation control element D-TLCE1 can comprise a translation control element derived from the human TNNT1, or a transcript sequence thereof, the second downstream translation control element D-TLCE2 can comprise a translation control element derived from the human DEFA5, or a transcript sequence thereof, and the third downstream translation control element D-TLCE3 can comprise a translation control element derived from the human FTL or the human RPS27, or a transcript sequence thereof.

In another exemplary embodiment, the first downstream translation control element D-TLCE1 can comprise a translation control element derived from the human albumin, or a transcript sequence thereof, the second downstream translation control element D-TLCE2 can comprise a translation control element derived from the human DEFA5, or a transcript sequence thereof, and the third downstream translation control element D-TLCE3 can comprise a translation control element derived from the human RPS27, or a transcript sequence thereof.

In another exemplary embodiment, the first downstream translation control element D-TLCE1 can comprise a translation control element derived from the human RPS27, or a transcript sequence thereof, the second downstream translation control element D-TLCE2 can comprise a translation control element derived from the human FTL, or a transcript sequence thereof, and the third downstream translation control element D-TLCE3 can comprise a translation control element derived from a gene selected from the group consisting of the human CCL19, the human RPS27 and the human DEFA5, or a transcript sequence thereof.

In another exemplary embodiment, the first downstream translation control element D-TLCE1 can comprises a translation control element derived from the human DEFA5, or a transcript sequence thereof, the second downstream translation control element D-TLCE2 can comprise a translation control element derived from the human TNNT1, or a transcript sequence thereof, and the third downstream translation control element D-TLCE3 can comprise a translation control element derived from the human FTL, or a transcript sequence thereof.

### Recombinant Expression Vector, Expression Construct

Each of the nucleic acid molecules illustrated in FIGS 1 and 2 can be inserted into a recombinant expression vector. The recombinant expression vector can comprise translation control element TLCE (*e.g*., U-TLCE and D-TLCE) with translation initiation activity, a coding region CR, and optionally, a transcription control element TCCE and/or a polyadenylation signal sequence PA. In other words, the recombinant expression vector can comprise the nucleic acid molecule with referring to FIGS. 1 and 2, which can be linked to other nucleic acids to encode a fusion protein or a fusion peptide.

For example, vectors can comprise viral vectors, DNA or RNA expression vectors, plasmid, cosmid or phage vectors, DNA or RNA expression vectors linked to CCA (cationic condensing agents), and specific eukaryotic cells such as producer cells.

As an example, the nucleic acid molecule of the present disclosure can be constructed to enter the mammal cells and to be expressed. Such construction can be particularly useful for using treatments and/or preventions of the infectious diseases. There are many methods for expressing nucleic acid molecules in the host cells and any appropriate methods can be used. For example, the nucleic acid molecule in accordance with the present disclosure can be inserted into any viral vectors such as adenovirus-based vectors, adeno-associated virus-based vectors, retrovirus-based vectors, vaccinia or other fox virus(*e.g*., avian pox virus)-based vectors.

The technologies for inserting the nucleic acid molecules, for example, DNA into such vectors are well known in the art. It is possible to insert additionally targeting moieties such as selection marker genes for making easy certification or selection for the transfected cells and/or genes encoding ligands acting as a receptor to a particular target cell in the retrovirus vector. Targeting may be performed by known processes using specific antibodies

It is possible to use plural vectors that are commercially available and known to in the art for the purposes of the present disclosure. Selecting appropriate vectors will be mainly dependent upon the sizes of the nucleic acid molecules to be inserted into the vectors and specific host cells transfected with the vectors. Each vector contains various components, depending upon its functions (amplification and/or expression of foreign polynucleotides) and compatibilities to the specific host cells having thereof. Vector components generally comprises, but are not limited to, replication origins (especially if the vector is inserted into prokaryotes), selection marker genes, promoters, ribosome binding sites (RBS), signal sequences, foreign nucleic acids insert, and a transcription termination sequence.

For example, the expression vector in accordance with the present disclosure can comprise expression control element, for example, an initiation codon, a termination codon, a polyadenylation signal sequence, an enhancer, a signal sequence for membrane targeting or secretions, and the likes that can influence on the expression of the protein and/or peptide (*e.g.,* reporter protein/peptide, antigen, therapeutic protein/peptide) encoded in the coding region CR.. The polyadenylation signal sequence PA makes transcript safety increase and facilitates cytoplasm transportation of the transcript. Enhancer sequences are nucleic acid sequences which are located at various sites with regard to transcription control sequence, e.g. promoter and increase transcription activity compared to a transcription activity by the promoter without the enhancer sequences.

Signal sequences comprise, but is not limited to, PhoA signal sequence, OmpA signal sequence, and the likes in case the host cell is bacteria in *Escherichia spp.,* α-amylase signal sequence, subtilisin sequence and the likes in case the host cell is bacteria in *Bacillus spp.,* MF-α signal sequence, SUC2 signal sequence and the likes in case the hose cell is yeast, and insulin signal sequence, α-interferon signal sequence, antibody molecule signal sequence and the likes in case the host cell is mammals.

A category of vectors is a 'plasmid' which refers to a circular, double-stranded DNA loop into which additional nucleic acid fragments can be ligated. Another category of vectors is a phage vector. Still another category of vectors is viral vectors into which additional nucleic acid fragments can be ligated into the viral genome. Specific vectors (*e.g*., viral vectors and episome mammalian vectors having bacterial replication origins) can replicate autonomously into the host cells having the transfected the vectors. Other vectors (*e.g*., non-episome mammalian vectors) can be integrated into the genome of a host cell as they transfect the host cell, and thereby, being replicated together with the genome of the host cell. Such vectors can be referred to "recombinant expression vector" (or in short "recombinant vector") herein. Generally, the expression vectors, which may be useful for recombinant DNA technologies, exist as a shape of plasmid.

As an example, the nucleic acid molecule can be inserted to the host cells using viral expression system (vaccinia or other pox viruses, retro virus or adenovirus). For example, the viral vector can comprise, but is not limited to, retrovirus derived from HIV, SIV, murine retroviruses, gibbon ape leukemia viruses, AAVs (adeno-associated viruses) and adenoviruses (Miller et al., Mol. Cell Biol. 10: 4328; J. Kolberg 1992, NIH Res. 4: 43; Cornetta et al., 1991, Hum. Gene Ther. 2: 214). Retrovirus vectors induced from murine leukemia virus (MuLV), gibbon ape leukemia virus (gaLV), ectotropic retrovirueses, SIV (simian immunodeficiency virus), and HIV (human immunodeficiency virus) hare widely used (Buchscher et al., 1992, J. Virol., 66(5): 2731-2739; Jonann et al., 1992, J. Virol. 66(5): 1635-1640; Sommerfelt et al., 1990, Virol. 176: 58-59; Wilson et al., 1989, J. Virol. 63: 2374-2378; Miller et al., 1991, J. Virol. 65: 2220-2224; Rosenberg and Fauci 1993 in Fundamental Immunology, Third Edtion, W. E. Paul (ed.) Raven Press Ltd., New York and the references therein; Miller et al., 1990, Mol. Cell. Biol. 10: 4239; R Kolberg 1992, J. NIH Res. 4: 43; Cornetta et al., 1991, Hum. Gen. Ther. 2: 215).

The vector system in accordance with the present disclosure can be constructed through various methods known in the relevant art, and the specific method are disclosed in Sambrook et al., Molecular Cloning, A Laboratory Manual, Cold Spring Harbor Laboratory Press (2001), which is incorporated herein by reference.

For example, the vector of the present disclosure can be construed for a typically cloning vector or expression vector. In addition, the vector of the present disclosure can be constructed by using prokaryotes or eukaryotes as the host cells. For example, the vector that can be utilized in the present disclosure can be fabricated by maneuvering usually used plasmid (*e.g.*, pSC101, ColE1, pBR322, pUC8/9, pHC79, pUC19, pET and the like), phage (*e.g.,* λgt4λB, λ-Charon, λΔz1, λGEM.TM.-11, M13 and the like), or virus (*e.g*., SV40 and the like).

Constitutively or inducible promoters (*e.g*., transcription control element) can be used in the present disclosure in accordance with specific environmental conditions that can be certified by an ordinary skilled person. Plural promoters that recognized by various possible host cells have been widely known in the art. Selected promoters may be operably linked to the nucleic acid molecule having the coding region CR comprising an open reading frame (ORF) of genes or transcripts encoding peptides and/or proteins by removing the promoters from supplier nucleic acid molecule through restriction enzyme digestions and then inserting the isolated promoter sequences into the selection vectors. It is possible to direct amplification and/or expression of the target genes using both natural promoter sequences and a plurality of foreign promoters. But, foreign promoters are generally more preferable to the natural targeting polypeptide promoters because the foreign promoters allows much transcription and high yield of the expressed target genes compared to the natural targeting polypeptide promoters.

For example, when the vector of the present disclosure is an expression vector and a host cell is eukaryotes, the vector may comprise the transcription control element TCCE such as strong promoters for proceeding with the transcription (e.g. tac promoter, lac promoter, LacUV5 promoter, lpp promoter, pLλ promoter, pRλ promoter, rac5 promoter, amp promoter, recA promoter, SP6 promoter, trp promoter and T7 promoter), the translation control element TLCE for initiating translation, and transcription/translation terminal sequence. In case of using *E. coil* as the host cell, promoters and operator sites of E. coli tryptophan biosynthetic pathway (Yanofsky, C., J. Bacteriol., 158:1018-1024, 1984), and pLλ promoter (Herskowitz, I. and Hagen, D., Ann. Rev. Genet., 14:399-445, 1980) can be used as a control site.

Alternatively, when the vector of the present disclosure is an expression vector and uses eukaryotes as the host cell, the vector may comprise promoters such as promoters derived from the genome of the mammalian cells (e.g., metallothionein promoter), promoters derived from mammalian viruses (e.g. adenovirus late promoter, vaccinia virus 7.5K promoter, SV40 promoter, cytomegalovirus promoter and tK promoter of HSV) or promoters derived from phages (e.g. T7 promoter, T3 promoter and SM6 promoter), and polyadenylation signal sequences PA as a transcription termination signal sequence.

Besides, when the recombinant vector of the present disclosure is a replicable repression vector, it may comprise a replication origin, which is a specific nucleic acid sequence for initiating replication. In addition, the recombinant vectors may comprise sequences encoding selectable markers. The selectable markers are intended to screen transfected cells by the vectors and markers giving selectable phenotypes such as drug resistances, nutritional requirements, cytotoxic agent resistances, or expressions of surface proteins may be used. The vectors of the present invention may comprise antibiotics resistant genes which have been conventionally used in the art, for example, ampicillin, gentamicin, carbenicillin, chloramphenicol, streptomycin, kanamycin, geneticin, neomycin, and tetracycline resistant genes as selectable markers. It is possible to screen the transfected cells because only cells expressing the selectable markers can survive in an environment of treating elective agents. Representative example of the selectable markers may comprise an auxotrophic marker, ura4, leu1, his3 and the likes, but the selectable markers can be used in the present invention is not limited to such an example.

Various *in vitro* amplification techniques that amplify sequences sub-cloned to expression vectors have been known. There are PCR (polymerase chain reaction), LCR (ligase chain reaction), Qβ-replicase amplification and techniques using other RNA polymerases in such techniques (Sambrook et al., 1989, Molecular Cloning-A Laboratory Manual (2nd Ed.) 1-3; U.S. Patent No. 4,683,202; PCR protocols: A guide to Methods and Applications, Innis et al. eds. Academic Press Inc., San Diego, CA 1990. Improved methods of cloning *in vitro* amplified nucleic acids are described in U.S. Patent No. 5,426,039).

The vector of the present disclosure may be fused with other sequences in order to facilitate the purification of the peptide expressed therefrom. Fused sequences comprises glutathione S-transferase (Pharmacia, USA), maltose binding protein (NEB, USA), FLAG (IBI, USA), 6 x His (hexahistidine, Quiagen, USA) and the likes, and 6 x His is most preferable. Owing to the additional sequences for purification, the proteins expressed in the host cell are purified with promptness and ease through affinity chromatography assay. If necessary, a sequence encoding Fc fragments may be fused with the vector in order to facilitate extracellular secretion of those peptides.

In accordance with an exemplary embodiment of the present disclosure, the peptides expressed by the vector which comprises the nucleic acid molecule are purified by affinity chromatography. For example, it is possible to purify the peptides or proteins of interest with promptness and with ease by using glutathione as a substrate of glutathione-S-transferase when glutathione-S-transferase is fused with the vector, and by using Ni-NTA His-binding resin column (Novagen, USA) when the vector comprises 6 x his.

It is possible to use any host cells known in the art as long as the host cells make the vectors stably and continuously clone and express. For example, host cells may comprise *E. coli* JM109, *E. coli* BL21 (DE3), *E. coli* RR1, *E. coli* LE392, *E. coli* B, *E. coli* X 1776, *E. coli* W3110, *Bacillus* strains such as Bacillus *subtilis, Bacillus thuringiensis,* and enterobacteriaceae strains such as *Salmonella typhimurium, Serratia marcescens* and various *Pseudomonas* species. Besides, yeast (*Saccharromyce cerevisiae*)*,* insect cells (e.g., SF9 cell), human cells (e.g., CHO (Chinese hamster ovary) cell, W138, BHK, COS-7, 293, HepG2, 3T3, RIN and MDCK cell lines) may be used as host cells in case of transfecting eukaryotes with the vectors of the present invention.

The vectors of the present disclosure may be used in modifying genetically the host cells in *in vivo* or *ex vivo* or in *in vitro.* With regard to methods of modifying genetically cells, various methods comprising cell transfection or transduction with viral vectors, calcium phosphate precipitation, methods of fusing recipient cells with bacterial protoplast containing DNA, method of treating recipient cells with liposome or microspheres containing DNA, DEAE dextran, receptor mediated endocytosis, electroporation, micro-injection, and gene bombardment are well known in the art.

For example, when the host cells are eukaryotes, the vector may be injected into the host cells by Hanahn method (Hanahan, D., J. Mol. Biol., 166:557-580, 1983) and/or electroporation method (Dower, W.J. et al., Nucleic. Acids Res., 16:6127-6145, 1988). In addition, when the host cells are eukaryotes, the vector may be injected into the host cells by micro-injection method (Capecchi, M.R., Cell, 22:479, 1980), calcium phosphate precipitate method (Graham, F.L. et al., Virology, 52:456, 11973), electroporation method (Neumann, E. et al., EMBO J., 1:841, 1982), liposome-mediated transfection method (Wong, T.K. et al., Gene, 10:87, 1980), DEAE-dextran treatment method (Gopal, Mol. Cell Biol., 5:1188-1190, 1985) and gene bombardment method (Yang et al., Proc. Natl. Acad. Sci., 87:9568-9572, 1990). The vector injected into the host cells may be expressed within the cell in which large amount of recombinant peptides or proteins are obtained. For example, when the expression vector includes lac promoter, it is possible to induce gene expression by treating IPTG to the host cells.

The expression construct of the nucleic acid molecule and/or the expression system in which the nucleic acid molecule is inserted can be utilized as a detection system for detecting any materials, a vaccine for inducing immune responses and/or therapeutics for treating diseases.

As an example, a pharmaceutical composition such as a vaccine or therapeutics including a pharmaceutically effective amount of the nucleic acid molecule or the expression construct having the nucleic acid molecule can comprise a pharmaceutically acceptable carrier, a diluent and/or an excipient/vehicle. As used herein, the term "pharmaceutically effective amount" means an amount of sufficiently accomplishing efficacy or activation of the nucleic acid molecule of the present disclosure.

In addition, such a pharmaceutical composition can further comprise a stabilizer such as a cationic polymer a cationic peptide or a cationic polypeptide that stabilize the nucleic acid molecule or the expression construct; at least one adjuvant that can enhancing immune responses; a sustained-release formulations; and/or a lipid nano particle (LNP) that can protect the nucleic acid molecule of an active ingredient and can improve injection activity of the nucleic acid molecule.

The nucleic acid molecule or the expression construct of the present disclosure can be administered by any appropriate means, for example, in oral, topical (including buccal and sublingual), rectal, vaginal, transdermal, parenteral, subcutaneous, intraperitoneal, intrapulmonary, intradermal, intrathecal, intradural, epidural, and if desired, by means for topical treatment, intralesional administration. Parenteral injection includes intramuscular, intravenous, intraarterial, intraperitoneal, or subcutaneous administration.

The nucleic acid molecules or expression constructs of the present disclosure can be administered in any convenient dosage form, such as tablets, powders, capsules, solutions, dispersions, suspensions, syrups, sprays, suppositories, gels, emulsions, patches, etc. These compositions may contain ingredients customary for pharmaceutical preparations, such as diluents, carriers, pH adjusters, sweeteners, bulking agents and further active agents.

Hereinafter, the present disclosure will be described by Examples, but the present disclosure is not limited to the following Examples.

### Comparative Example 1: Preparation of Nucleic Acid Molecule Having Inserted Encoding Renilla Luciferase (R/L)

A nucleic acid molecule into which nucleotides encoding Renilla Luciferase was prepared. The template DNA having a coding region encoding the Renilla Luciferase was designed as follows:

5'- KpnI recognition sequence (GGTACC) - T7 promoter (SEQ ID NO: 16) - upstream translation control sequence derived human troponin T1 (TNNT1) (SEQ ID NO: 1) - PacI recognition sequence (TTAATTAA) - Kozak sequence (GCCACC) - Renilla Luciferase encoding sequence (R/L, SEQ ID NO: 15) - ClaI recognition sequence (ATCGAT) - downstream translation control sequence derived from human troponin T1 (TNNT1) (SEQ ID NO. 8) - EcoRI recognition sequence (GAATTC) - polyadenylation signal (SEQ ID NO: 17) - restriction endonuclease recognition sequence (SEQ ID NO: 19)

The template DNA was inserted into a downstream of a pGH vector (SEQ ID NO: 18), cloned and linearized by restriction endonuclease through *in vitro* transcription (IVT) so that the nucleic acid molecule of RNA platform type (hereinafter, "pHJ5L) was prepared.

### Example 1: Preparation of Nucleic Acid Molecule Having Inserted Encoding Renilla Luciferase (R/L)

Nucleic acid molecules were prepared as the same process as Comparative Example 1, except two downstream translation control sequences are inserted into the downstream of the coding region encoding the Renilla Luciferase instead of the single translation control element derived TNNT1. Each of two downstream translation control elements is independently selected from the group consisting of SEQ ID NO. 8 (derived from human TNNT1), SEQ ID NO. 9 (derived from human albumin), SEQ ID NO. 10 (derived human FTL), SEQ ID NO: 11 (derived from human CCL19), SEQ ID NO. 12 (derived from human AAMP), SEQ ID NO: 13 (derived from human RPS27) and SEQ ID NO: 14 (derived from human DEFAS). Hereinafter, the downstream translation control element of SEQ ID NO: 8 is referred to "A", the downstream translation control element of SEQ ID NO: 9 is referred to "B", the downstream translation control element of SEQ ID NO: 10 is referred to "C", the downstream translation control element of SEQ ID NO: 11 is referred to "D", the downstream translation control element of SEQ ID NO: 12 is referred to "E", the downstream translation control element of SEQ ID NO: 13 is referred to "F", and the downstream translation control element of SEQ ID NO: 14 is referred to "G".

### Experimental Example 1: In vitro Expression

Each of Nor 10 cells of mouse muscle cell line, HeLa cells of proliferation cell line derived from human and 293A cells of human embryo kidney cell line were inoculated to a 48-well plate with a concentrations of 8 ×10⁴ cells/well, respectively. The plate was placed into 37°C incubator to allow the cells to attach to the plate and grow for 24 hours. Each of the mRNAs prepared in Comparative Example and Example was transfected to each of the cell lines with a concentration of 500 ng/well. Lipofectamine 2000^{™} was used at 1 µl per 1 µg of each mRNA and treated in an OPTI-MEM medium for more than 30 minutes to ensure sufficient mixing. When the mRNA and Lipofectamine 2000^{™} were mixed, the 48-well plate with the attached cells was taken out, the supernatant was removed, washed with PBS, and the supernatant was removed again. 100 ul of medium was added on the cells, and 100 ul of a mixture of mRNA and Lipofectamine 2000^{™} was added.

At this time, only 200 ul medium was added to the negative control group. The plate was placed back in the 37°C incubator and was taken out at 6 and 24 hours later to confirm expression. When 6 and 24 hours have elapsed, the plate was taken out, all supernatant was removed, the plate was washed once with PBS, and 80 ul of Renilla lysis buffer from the Renilla Luciferase assay system kit (Promega) was added on the cells to sufficiently destroy the cells. After doing so, the expression level of Renilla Luciferase was measured by spectrophotometer according to the kit instructions.

The expression level of Renilla Luciferase after 6 hours in the Nor10 cell line to which the pHJ5L nucleic acid molecule prepared in the Comparative Example was applied was 2×10⁷ and the expression level of Renilla Luciferase after 24 hours was 2×10⁶. The expression level of Renilla Luciferase after 6 hours in the HeLa cell line to which the pHJ5L nucleic acid molecule prepared in the Comparative Example was applied was 7×10⁷, and the expression level of Renilla Luciferase after 24 hours was 5.5×10⁶. The expression level of Renilla Luciferase after 6 hours in the 293A cell line to which the pHJ5L nucleic acid molecule prepared in the Comparative Example was applied was 4.3×10⁷, and the expression level of Renilla Luciferase after 24 hours was 2.2×10⁶.

The nucleic acid expression platform that serves as the basic template is pHJ5L prepared in Comparative Example, and the expression level of Renilla Luciferase in this platform is set to 1, and the expression level of Renilla Luciferase in the RNA expression platform prepared in Example 1 was compared with the expression level in pHJ5L. The measurement results are shown in FIGS. 3 to 8. As shown in FIGS. 3 to 8, the nucleic acid molecules prepared in the Example in which two downstream translation control elements were inserted between the coding region and the 3' terminus allowed the expression levels of Renilla Luciferase that were inserted into the coding region to be improved efficiently in all of the Nor10 cell line, HeLa cell line, and 293A cell line.

### Experimental Example 2: In vivo Expression

The mRNAs prepared in Comparative Example and Example 1 was formulated with LNP (the same as that used in the Moderna vaccine) to prepare a composition for injectable living organisms. The mouse was anesthetized through respiratory anesthesia and its ears were fixed. The composition containing the mRNAs prepared in Comparative Examples and Example 1 was injected into each mouse ear at a concentration of 5 µg/20 µl. At 6 and 24 hours after injection, the mice were sacrificed, their ears were cut, placed in 300 ul of Renilla lysis buffer, and cut into small pieces with scissors. The cut mouse ears were further finely ground using a homogenizer, and 20 ul of the supernatant was placed in a white 96-well plate. Afterwards, luminescence was measured with a spectrophotometer using the Renilla substrate in the Renilla luciferase assay kit. The nucleic acid expression platform that serves as the basic template is pHJ5L prepared in the Comparative Example, and the expression level of Renilla Luciferase in this platform is set to 1, and the expression level of Renilla Luciferase in each RNA expression platform prepared in Examples was compared with the expression level in pHJ5L. The measurement results are shown in FIGS. 9 and 10. As shown in FIG. 9 and 10, the expression level of Renilla Luciferase was increased in the nucleic acid molecule in which two downstream translation control elements were inserted between the coding region and the 3' terminus.

### Example 2: Preparation of Nucleic Acid Molecule Having Inserted Encoding Renilla Luciferase (R/L)

Nucleic acid molecules are prepared as the same process as Example 1, except three downstream translation control sequences are inserted into the downstream of the coding region encoding the Renilla Luciferase instead of two downstream translation control sequences. Each of three downstream translation control elements were independently used the same downstream translation control element as Example 1.

### Experimental Example 3: In vitro Expression

The nucleic acid molecules prepared Example 2 were transfected into the 293 A cell line of a human embryonic kidney cell line, in the same manner as in Experimental Example 1, and the expression level of Renilla Luciferase was measured by luminescence using a spectrophotometer. The nucleic acid expression platform that serves as the basic template is pHJ5L prepared in Comparative example, and the expression level of Renilla Luciferase in this platform is set to 1, and the expression level of Renilla Luciferase in the RNA expression platforms prepared in Example 2 is compared to the expression level in pHJ5L. The measurement results are shown in FIGS. 11 and 12. As shown in FIGS. 11 and 12, the nucleic acid molecules prepared in Example 2 in which three downstream translation control elements were inserted between the coding region and the 3' terminus efficiently improved expression levels of Renilla Luciferase inserted into the coding region in the 293A cell line.

While the present disclosure has been described with reference to exemplary embodiments and examples, the present disclosure is not limited to those embodiments and examples. Rather, it will be apparent to those skilled in the art that various modifications and variations can be made in the present disclosure without departing from the scope of the disclosure. However, it is apparent that such modifications and variations are within the scope of the present disclosure from the appended claims.

## Claims

1. A nucleic acid molecule comprising:
a coding region; and
a translation control element operably linked to the coding region,
wherein the translation control element comprises an upstream translation control element located upstream of the coding region and a downstream translation control element located downstream of the coding region, and
wherein the downstream translation control element comprises plural downstream translation control elements.

2. The nucleic acid molecule of claim 1, wherein the downstream translation control element comprises a first downstream translation control element and a second downstream translation control element each of which is located at the downstream of the coding region.

3. The nucleic acid molecule of claim 1, wherein each of the plural downstream translation control elements independently comprises a translation control element derived from a gene selected from the group consisting of a human troponin T1 of slow skeletal type (human TNNT1), a human albumin, a human ferritin light chain (human FTL), a human C-C motif chemokine ligand (human CCL19), a human associated migratory cell protein (human AAMP), a human ribosomal protein S27 (human RPS27) and a human defensing alpha 5 (human DEFA5), or a transcript sequence thereof.

4. The nucleic acid molecule of claim 1, wherein each of the plural downstream translation control elements independently comprises a translation control element selected from the group consisting of SEQ ID NO: 8, SEQ ID NO: 9, SEQ ID NO: 10, SEQ ID NO: 11, SEQ ID NO: 12, SEQ ID NO: 13 and SEQ ID NO: 14, or a transcript sequence thereof.

5. The nucleic acid molecule of claim 3, wherein the first downstream translation control element comprises a translation control element derived from the human TTNT1, or a transcript sequence thereof, and wherein the second downstream translation control element comprises a translation control element derived from a gene selected from the group consisting of the human albumin, the human FTL, the human CCL19, the human AAMP, the human RPS27 and the human DEFA5, or a transcript sequence thereof.

6. The nucleic acid molecule of claim 3, wherein the first downstream translation control element comprises a translation control element derived from the human albumin, or a transcript sequence thereof, and wherein the second downstream translation control element comprises a translation control element derived from the human DEFA5, or a transcript sequence thereof.

7. The nucleic acid molecule of claim 3, wherein the first downstream translation control element comprises a translation control element derived from the human FTL, or a transcript sequence thereof, and wherein the second downstream translation control element comprises a translation control element derived from a gene selected from the group consisting of the human TNNT1, the human FTL, the human RPS27 and the human DEFA5, or a transcript sequence thereof.

8. The nucleic acid molecule of claim 3, wherein the first downstream translation control element comprises a translation control element derived from the human CCL19, or a transcript sequence thereof, and wherein the second downstream translation control element comprises a translation control element derived from a gene selected from the group consisting of the human TNNT1, the human FTL, the human CCL19, the human RPS27 and the human DEFA5, or a transcript sequence thereof.

9. The nucleic acid molecule of claim 3, wherein the first downstream translation control element comprises a translation control element derived from the human AAMP or a transcript sequence thereof, and wherein the second downstream translation control element comprises a translation control element derived from a gene selected from the group consisting of the human albumin, the human FTL, the human RPS27 and the human DEFA5 or a transcript sequence thereof.

10. The nucleic acid molecule of claim 3, wherein the first downstream translation control element comprises a translation control element derived from the human RSP27 or a transcript sequence thereof, and wherein the second downstream translation control element comprises a translation control element derived from a gene selected from the group consisting of the human TNNT1, the human albumin, the human FTL, the human CCL19, the human AAMP, the human RPS27 and the human DEFA5 or a transcript sequence thereof.

11. The nucleic acid molecule of claim 3, wherein the first downstream translation control element comprises a translation control element derived from the human DEFA5 or a transcript sequence thereof, and wherein the second downstream translation control element comprise a translation control element derived from a gene selected from the groups consisting of the human TNNT1, the human albumin, the human FTL, the human CCL19, the human AAMP and the human RPS27 or a transcript sequence thereof.

12. The nucleic acid molecule of claim 3, wherein the downstream translation control element further comprises a third downstream translation control element located downstream of the second downstream translation control element.

13. The nucleic acid molecule of claim 12, wherein the third downstream translation control element comprises a translation control element derived from a gene selected from the group consisting of a human troponin T1 of slow skeletal type (human TNNT1), a human albumin, a human ferritin light chain (human FTL), a human C-C motif chemokine ligand (human CCL19), a human associated migratory cell protein (human AAMP), a human ribosomal protein S27 (human RPS27) and a human defensing alpha 5 (human DEFA5), or a transcript sequence thereof.

14. The nucleic acid molecule of claim 13, the first downstream translation control element comprises a translation control element derived from the human TNNT1, or a transcript sequence thereof, wherein the second downstream translation control element comprises a translation control element derived from the human DEFA5, or a transcript sequence thereof, and wherein the third downstream translation control element comprises a translation control element derived from the human FTL or the human RPS27, or a transcript sequence thereof.

15. The nucleic acid molecule of claim 13, wherein the first downstream translation control element comprises a translation control element derived from the human albumin, or a transcript sequence thereof, wherein the second downstream translation control element comprises a translation control element derived from the human DEFA5, or a transcript sequence thereof, and wherein the third downstream translation control element comprises a translation control element derived from the human RPS27, or a transcript sequence thereof.

16. The nucleic acid molecule of claim 13, wherein the first downstream translation control element comprises a translation control element derived from the human RPS27, or a transcript sequence thereof, wherein the second downstream translation control element comprises a translation control element derived from the human FTL, or a transcript sequence thereof, and wherein the third downstream translation control element comprises a translation control element derived from a gene selected from the group consisting of the human CCL19, the human RPS27 and the human DEFA5, or a transcript sequence thereof.

17. The nucleic acid molecule of claim 13, wherein the first downstream translation control element comprises a translation control element derived from the human DEFA5, or a transcript sequence thereof, wherein the second downstream translation control element comprises a translation control element derived from the human TNNT1, or a transcript sequence thereof, and wherein the third downstream translation control element comprises a translation control element derived from the human FTL, or a transcript sequence thereof.

18. The nucleic acid molecule of claim 1, wherein the upstream translation control element comprises a translation control element derived from a gene selected from the group consisting of a human troponin T1 of slow skeletal type (human TNNT1), a human albumin, a human ferritin light chain (human FTL), a human C-C motif chemokine ligand (human CCL19), a human associated migratory cell protein (human AAMP), a human ribosomal protein S27 (human RPS27) and a human defensing alpha 5 (human DEFA5), or a transcript sequence thereof.

19. The nucleic acid molecule of claim 1, wherein the upstream translation control element comprises a translation control element selected from the group consisting of SEQ ID NO: 1, SEQ ID NO: 2, SEQ ID NO: 3, SEQ ID NO: 4, SEQ ID NO: 5, SEQ ID NO: 6 and SED ID NO: 7, or a transcript sequence thereof.

20. The nucleic acid molecule of claim 1, wherein the nucleic acid molecule is RNA.

21. The nucleic acid molecule of claim 1, wherein the nucleic acid molecule further comprises a transcription control element operably linked to the coding region.

22. The nucleic acid molecule of claim 1, wherein the nucleic acid molecule further comprises a polyadenylation signal sequence or a polyadenosine sequence located downstream of the downstream translation control element.

23. The nucleic acid molecule of claim 1, wherein the coding region encodes at least one of a reporter protein, a marker, a selection protein or a fragment thereof.

24. The nucleic acid molecule of claim 1, wherein the coding region encodes an antigen or a fragment thereof.

25. The nucleic acid molecule of claim 24, wherein the antigen comprises a peptide of a pathogenic antigen, a tumor antigen, or a variant or a derivative thereof.

26. The nucleic acid molecule of claim 1, wherein the coding region encodes a peptide for disease treatment or a fragment thereof.

27. A recombinant expression vector into which the nucleic acid molecule of claim 1 is inserted.

28. A process of producing a protein or a peptide, the process comprising:
injecting the nucleic acid molecule of claim 1 or a recombination expression vector into which the nucleic acid molecule of claim 1 is inserted into a host; and
expressing a protein or a peptide from the injected nucleic acid molecule in the host.
